# EUROPEAN PATENT SPECIFICATION

(11) **EP 4 326 157 B1**
(45) Date of publication and mention of the grant of the patent: **28.10.2026**
(21) Application number: 22790603.9
(22) Date of filing: 19.04.2022
(51) Int. Cl.: A61B 8/08, A61B 5/00, A61B 17/34, A61B 8/06, A61B 8/00

(54) **A PORTABLE ULTRASOUND DEVICE AND METHOD FOR ULTRASONIC IMAGING**
TRAGBARE ULTRASCHALLVORRICHTUNG UND VERFAHREN ZUR ULTRASCHALLBILDGEBUNG
DISPOSITIF À ULTRASONS PORTABLE ET PROCÉDÉ POUR IMAGERIE ULTRASONORE

(30) Priority: 19.04.2021 AU 2021901145
(43) Date of publication of application: 28.02.2024
(73) Proprietor: Veintech Pty Ltd, Osborne Park, WA 6017 (AU)
(72) Inventor: BAPPOO, Nikhilesh, Dianella, Western Australia 6059 (AU); BUCKLEY, Nicholas Michael, Dianella, Western Australia 6059 (AU); ARENSON, Katherine Jane, Dianella, Western Australia 6059 (AU); MEHTA, Ritu Sanjaybhai, Dianella, Western Australia 6059 (AU); AL ODEH, Saleem Ahmed Saleem, Dianella, Western Australia 6059 (AU)
(74) Representative: FRKelly
(86) International application number: PCT/AU2022/050356
(87) International publication number: WO 2022/221913

(56) References cited:
- EP-A1- 3 513 732
- WO-A1-2019/232451
- WO-A1-2019/232451
- US-A1- 2006 184 029
- US-A1- 2010 312 120
- US-A1- 2016 007 956
- US-A1- 2017 332 995
- US-A1- 2017 332 995
- US-A1- 2020 230 391

## Description

### TECHNICAL FIELD

The present invention relates to a portable ultrasound device for non-invasively imaging a selected sub-cutaneous structure and a method for non-invasively imaging a selected sub cutaneous structure.

### BACKGROUND ART

Peripheral intravenous cannulation (PIVC; placing a vascular access device, typically a plastic cannula, inside a vein with the help of an introducing needle) is the most commonly performed invasive medical procedure with over two billion cannulas sold worldwide each year (Rickard et al. (2018) Lancet 392(10145): 419-430). Over 70% of admitted patients will require a cannula during their admission (Zingg and Pittet (2009) Int J Antimicrob Agents 34 Suppl 4: S38-42).

The overwhelming majority of vascular access procedures using vascular access devices, such as cannulas, are performed without the aid of any visualisation device and rely on what is observed through the patient's skin and by the clinician's ability to feel the vessel. A patients' initial cannula is most commonly placed in the Emergency Department as this is the entry point for most admitted patients. These are often inserted in a rushed manner, involving guesswork in feeling or seeing veins, that results in a sub-optimal insertion location and method. Once the patient has stabilized, these PIVCs often need to be removed and reinserted.

Cannulas can be sited by doctors or nurses and this differs from country to country and hospital to hospital. In Western Australian public hospitals, the majority of cannulas are placed by junior doctors (Interns, Resident Medical Officers and Registrars) or trained clinical nurses.

When a cannulation is deemed difficult or there have been multiple failed attempts, junior doctors will enlist the help of senior doctors to reattempt the insertion. If multiple standard attempts fail then an appropriately trained, usually senior doctor, may use ultrasound to visualise a vein and guide further cannulation attempts. Ultrasound assisted cannulation or venepuncture requires significant training and experience and only a few departments have access to an ultrasound machine.

A major hurdle for poor ultrasound adoption is the difficulty in using the technology.

Firstly, end users require extensive training to adjust ultrasound settings and adequately position the hardware (probe) on the patient in order to obtain a clear image in the first place, let alone interpreting the images.

Secondly, it is difficult for end users, particularly those that perform most cannulations (junior doctors or accredited nurses) to objectively interpret traditional B-mode or colour doppler images and to identify and discriminate between arteries or veins to target.

Thirdly, usability issues exist, including the screen typically being on a separate cart-based system and not aligned with the position of blood vessels, therefore requiring end users to observe an adjacent screen while holding an imaging probe and trying to insert a needle.

According to the literature, cannulation fails an unacceptable 40% of the time on first attempt (Rickard et al. (2018) Lancet 392(10145): 419-430; Cooke et al. (2018) PLoS One 13(2): e0193436; Keogh and Mathew (2019) Australian Commission on Safety and Quality in Health Care*).*

Multiple cannulation attempts are often required in patients with difficult venous access. Not only do these multiple attempts cause considerable pain and distress to the patients, but they also cause frustration to the clinicians and significant time and equipment wastage. This causes downstream delays to investigations and treatments. These delays also slow the flow of patients through the department increasing emergency wait times.

Repeated cannulation attempts are also associated with higher infection rates, with increased morbidity and longer length of stays. This leads to increased costs to the health service (Bahl et al. (2016) The American Journal of Emergency Medicine, 34(10): 1950-1954; Fields et al. (2013) Journal of Vascular Access 15(6): 514-518).

First pass cannulation attempts may fail in patients for a number of reasons. Some may be related to patient anxiety and consequent movement, more may be related to the lack of skill or experience of the clinician, however the majority are because a patient has difficult venous access, reducing vein identification visually or by feel. By way of non-limiting examples, this may be as a result of obesity, past or current chemotherapy, previous intravenous drug use or renal failure and cardiac failure.

Other patients that can be difficult to cannulate include, but are not limited to, the very young and the very old, as well as patients with dark skin (Au et al. (2012) Am J Emerg Med 30(9): 1950-1954; Bauman et al. (2009) Am J Emerg Med 27(2): 135-140; Brannam et al. (2004) Acad Emerg Med 11(12): 1361-1363; Chinnock et al. (2007) J Emerg Med 33(4): 401-405). Obesity is by far the most common cause for difficult vascular access, with 28% of Australians and 13% of people worldwide being obese (World Health Organisation (2018)).

US2016007956 describes imaging devices for identification of target regions.

The previous discussion of the background art is intended to facilitate an understanding of the present invention only. The discussion is not an acknowledgement or admission that any of the material referred to is or was part of the common general knowledge as at the priority date of the application.

### SUMMARY

The present invention, as describes by the claims, provides for a portable ultrasound device for imaging a sub-cutaneous structure in a subject, for example, for assisting a practitioner in the performance of a venepuncture or cannulation.

In one aspect, the present invention provides a portable ultrasound device for non-invasively imaging a selected sub-cutaneous structure in a subject, comprising
(a) a housing;
b) a plurality of arrays of transducer elements, each array being arranged in parallel and each transducer element comprising a transmitter transducer and a receiver transducer, located within said housing for continuously transmitting ultrasound energy in a predetermined frequency range toward a body of a subject and continuously receiving echo signals in a predetermined frequency range from the body of the subject following reflection of ultrasound energy, said plurality of parallel arrays of transducer elements enabling imaging of the sub-cutaneous structure in multiple transverse and lateral planes;
(c) a controller for operating said plurality of parallel arrays of transducer elements and communicable with a processor for processing said received echo signals from said plurality of parallel arrays of transducer elements; and
(d) a screen forming part of said housing for displaying an interpretable image of said sub-cutaneous structure wherein said processor is configured to process said echo signals reflected from the sub-cutaneous structure to selectively produce said interpretable image of the sub-cutaneous structure of the subject,

wherein the controller is configured to operate said plurality of parallel arrays of transducer elements in continuous wave doppler mode; and
wherein said plurality of parallel arrays of transducer elements are spaced apart from each other by a distance ξ along a horizontal axis selected to minimise interference and maximise scanning window, wherein ξ is between 5 and 30mm.

It will be understood that the subject may be a human patient or a veterinary subject such as domestic animal, farm animal or laboratory animal

The portable ultrasound device is configured to facilitate operation by personnel without a specific specialisation in ultrasonic imaging. Typically, ultrasound operators have been required to manually adjust the probe's orientation, manually calibrate the operating field of view, gain levels, contrast levels, imaging depth or doppler parameters all while interpreting an abstract 2D grayscale image (or 2D colour-doppler) taken in a single scan line. The device is conveniently configured, as described below, or the controller of the portable ultrasound device is conveniently programmed to undertake one, more or all of these functions to avoid need for a user of the device to do so. For example, gain control may be made completely automatic without the user of the portable ultrasound device requiring to adjust gain. The portable ultrasound device may simply be provided with user functions that allow the device to be powered up and down and to select a favoured image mode, for example and preferably between B mode, colour Doppler mode and a schematic mode enabling display of a sub-cutaneous structure, for example a vascular structure. The user functions may, if desired, include a function to store an image as described below. In addition, in embodiments of the portable ultrasound device as described below, there is no need to manually adjust the probe orientation.

The sub-cutaneous structure is preferably a vascular structure, such as a vein or artery. However, the portable ultrasound device may be configured to alternatively, or additionally, enable imaging of other sub-cutaneous structures for nerve imaging and/or foreign body imaging.

Planar or linear arrays of transducer elements are desirable. The plurality of arrays of transducer elements may include any convenient number of arrays, say four arrays. Preferably, the plurality of arrays of transducer elements are spaced apart from each other by a distance ξ along the horizontal axis chosen to minimise interference and maximise scanning window. Most preferably, ξ is between 5 and 30mm.

The arrays of transducer elements preferably comprise at least one Doppler transducer array. Preferably, the parallel transducer arrays are angled at an angle of insonation Φ where 10<Φ<60 to ensure the Doppler effect is captured and not nullified. Such an angled configuration also allows removal of need for an operator to manually adjust an operating field of view and imaging angles.

The plurality of parallel transducer arrays provides the ability to image the sub-cutaneous structure in multiple transverse and lateral planes to display its lateral position, up to a selected depth, for example 2-3 cm, below the skin surface in a contacting area where the ultrasound device contacts the skin of the subject. By providing simultaneous transverse and lateral imaging, images are effectively provided with a '3D' appearance making the image easier to interpret, and use in effective cannulation, whether by personnel trained or untrained in ultrasonic imaging.

A linear array may be singular and affixed to a mobile track apparatus that allows the transducer array to move up the length of the vessel while being used to sense multiple ultrasound images for processing. Movement of the array on a track allows for imaging to occur in multiple planes, allowing for the combined lateral and transverse imaging mentioned previously. For example, rather than having say 4 crystal arrays in a fixed position, a single moveable array can achieve the same multiplanar function

A linear transducer may be single and oscillate around a fixed point whilst sensing multiple ultrasound images for processing.

The controller of the ultrasound device controls operation of the plurality of arrays of transducer elements to selectively transmit ultrasound energy to locate the sub-cutaneous structure which is then displayed on the screen following processing of the received echo signals by the processor. Where vascular structures are concerned, and vascular structures such as veins are described below for purposes of exemplification, candidate sub-cutaneous vascular structures are conveniently located in both transverse and lateral directions. This may involve transmitting ultrasound energy to the body of the subject to a selected depth of from less than 1 cm, with a dead zone about 5 mm, up to several centimetres, for example up to about 5 cm for a relatively narrow frequency range of 3-8 MHz, at which a candidate vascular structure is located. Transmission of ultrasound energy to greater depth is prevented to avoid receiving echo signals not reflective of a vascular structure and not of assistance in preparation for a cannulation procedure with a vascular access device such as a cannula or needle. This may involve, or also involve, processing echo signals to remove noise signals not reflective of candidate vascular structures. The processor enables updated display on the screen of the path of the venous structure as the portable ultrasound device is moved over the skin surface. The image quality is suitable for locating a suitable cannulation point to assist venepuncture

In preferred embodiments, the one-dimensional position (as an x-axis coordinate) of veins and arteries are identified across a singular transducer through a number of possible methods described below. This process advantageously occurs in real time for the plurality of transducers in the array and x-axis positions for each vein or artery locations can be stored and can be interpolated between transducers to create a venous or arterial path.

Advantageously, where sub-cutaneous vascular structures are of interest, the processor is programmed with instructions to discriminate between arterial and venous vascular structures. In an embodiment, a first algorithm allows such discrimination with an option being a time-domain based algorithm based on measurement of pulsatility. Veins are typically far less pulsatile than arteries, if pulsatile at all. Pulsatility metrics suitable for measurement and input to such an algorithm may include pulsatility index (i.e. (systole-diastole)/mean blood velocity in vessel), dicrotic notch, velocity reflection index (VRI), viscosity elastic index (VEI) and/or resistance index.

In another embodiment, the processor may be programmed with instructions to identify and discriminate between arterial and venous structures using an energy signal or energy signature to determine the position of a vein below the contacting area of the ultrasound device. The energy signal is preferably determined from a frequency-domain representation of the raw signal, determined by a Fast Fourier Transform (FFT) of the continuously acquired current signal or more preferably from a power spectral density (PSD) computed from the sampled signal. This energy signature is preferably the amplitude of the dominant signal frequency, or alternatively the sum of amplitudes of dominant frequencies or else the sum of amplitudes squared. More preferably the energy is the area under the FFT or PSD distribution. Alternatively, the energy could be the ratio of amplitude to area under the distribution or the ratio of sum of amplitudes (or sum of amplitudes squared) to the area under the distribution.

The processor may also be programmed with instructions to discriminate between veins and arteries due to intrinsic differences in the Doppler shift measured. If blood flow in veins is towards the transducer while the opposite occurs for arteries, the frequency shift can be either positive (veins) or negative (arteries). The converse is true if vein flow is away from the transducer and artery flow is towards the transducer. According to the directionality inferred from the Doppler Shift, the processor may determine a pseudo-negative magnitude for arteries, which also typically has a larger absolute magnitude than that of the vein. As such, veins and arteries can be discriminated.

The processor may also determine depth of the sub-cutaneous structure below the skin. For example, in the case of vascular structures, for a given vessel diameter, which may be calculated by the processor using a method such as those described below, characteristic curves may be inferred relating magnitude of energy to the vessel depth for different vessel diameters. This depth can be visualised numerically as a value in millimetres or centimetres below the skin, or through colour weightings by depth magnitude or through other visual representations such as 3D perception of shallowness or depth, all of which may conveniently be displayed on the screen. Thus, the processor may calculate depth without the need for B-mode cross sectional image reconstructions.

Alternatively, the processor may be programmed within instructions to perform B-mode imaging for each transducer element. Structural metrics (such as diameter or dimension, compressibility, and other curve features) can be calculated and used to discriminate between arteries and veins as described below. Machine learning techniques, for example including the use of convolutional neural networks, can be used to classify and identify veins and arteries, following training datasets provided by a population of ultrasound users. In B-mode or colour Doppler representations, depth of vessels can be directly measured and displayed on the screen of the ultrasound device.

In another embodiment of an algorithm for artery or vein discrimination, B-mode and Colour Doppler images are acquired and automatic computer vision techniques may be used to identify flow away or towards the transducer (or flow away from the heart, representing arteries, and flow towards the heart, representing veins). Colour Doppler represents flow towards the transducer (veins typically) as shades of blue with intensities representing velocity magnitudes, and flow away from the transducer (arteries typically) as shades of red. As such, arteries or veins can be distinguished through automatic computer vision techniques discriminating colour. In B-mode or colour Doppler representations, depth of vessels can be directly measured and displayed.

Conveniently, where energy signals are captured, the algorithm - as embodied in instructions programmed in the processor - recognizes that the peak of the energy signal or energy signature, forms part of parabolic or gaussian resembling distributions and that the start and end points of such distributions can be used to estimate structure sub-cutaneous dimension or diameter, particularly in the lateral direction.

Ultrasound data may be acquired by the processor for processing without and with mechanical compression of the subcutaneous vessels through application of external force by the housing of the ultrasound device onto the skin. Taking advantage of different vascular wall properties of veins (compressible but not muscular and not elastic) and arteries (muscular and elastic), changes during compression can be measured to distinguish between arteries and veins.

In one embodiment of the compression method, peak energy signals (determined from either FFT or PSD) change with compression. Notably, the positive peak (representing a flow in a vein when flowing towards the transducer) is flattened with compression while the negative peak (representing a flow in an artery when flowing away from the transducer), only shows reductions in magnitude while maintaining its directionality (negative peak representing flow away from the transducer). The characteristic drop in the peak (toward zero) for the vein and a linear decrease in magnitude for the artery are preferably calculated through absolute value measurements or more preferably through feature curve analysis to discriminate between arteries and veins. Alternatively, as vessel diameter can be determined from energy curves as previously described, the change in size due to compression can be estimated by calculating the change in diameter from before compression to after compression. A large deformation (say equivalent to 90 - 100% of the original diameter) is expected for veins compared to arteries. Preferably, the method is applicable to sub-cutaneous structures, i.e. veins or arteries, up to 5 cm in depth but most preferably up to 2-3 cm in depth. Vessel elasticity may also be determined.

In another embodiment of the compression method utilizing B-mode or colour doppler, automatic feature edge recognition is used to identify circular or elliptical objects and the maximum vertical chord length of said objects can be measured. Circular or elliptical objects can be identified as veins or arteries as the reduction in maximum vertical chord length is largest for veins (say equivalent to 90 - 100% of the original diameter) compared to arteries.

In another embodiment of both compression methods described, the compressibility of the vein either through energy drops or chord length drops can be used to indicate the structural integrity of the vein with larger resistance to compression indicating higher stiffness (structural integrity). Additionally, the restoration of structure following compression can be used to determine the extent of plastic deformations and therefore can be used to determine elasticity. The choice of structurally stable veins through both stiffness and elasticity inferences is a critical factor for cannula insertions as well as cannula success throughout indwelling periods (no dislodgement, extravasation).

In another embodiment, where both diameter and path of a vascular structure is available to the processor, the processor may be programmed with instructions to create a three dimensional structure of that vascular structure. The path data is used as a centreline and diameter data for each point may be used to create idealized circular cross sections with constant diameter for the energy based algorithm approach) or subject specific vascular cross sections which may or may not be circular (for B-mode or colour doppler approach). These cross sections can then be lofted along the vessel path to create three dimensional structures, with triangulated surfaces created by algorithms such as marching cubes.

The processor desirably includes a graphics processor which assists in providing requisite image quality in terms of providing sufficient information to identify the candidate vascular structure without reduction to a simple schematic which provides too little information to assist with cannulation.

Processing of received echo signals during ultrasound investigation involves numerous complex calculations which typically makes medical ultrasound equipment non-portable due to the wide range of ultrasound investigations that such equipment is called upon to perform. With the present procedure restricted, in preferred embodiments, to locating candidate vascular structures for cannulation, this complexity reduces but is still present. To that end, the processor - though it is desirably accommodated within the housing - may involve processing units located outside the housing but communicable through wire or wireless network with the portable ultrasound device to enable display of a candidate vascular structure on the screen forming part of the housing of the portable ultrasound device.

Processing involving processing units located outside the housing, if necessary because inclusion of the processor within the housing of the device is most preferred, may be performed using a cloud-based system such as Amazon AWS, whereby data are wirelessly transmitted, processed and returned to the device. Preferably, trivial and junk signals are automatically excluded while Doppler frequencies are calculated for non-trivial signals and stored on the temporary sampling memory. Preferably, the temporary sampling memory will be less than 8GB, more preferably less than 6GB or most preferably less than 4GB or 2 GB. Wireless communications from portable ultrasound device to processing units or storage devices may be made, for example, by means of reversible USB-C, Bluetooth 5.0, or wireless internet in the form of standard WiFi, dual-band, Wi-Fi Direct or hotspot. In remote usage, in the absence of wireless internet, the portable ultrasound device may have standard GSM or CDMA or HSPA or EVDO or LTE network technology paired with single SIM (nano SIM).

The housing of the portable ultrasound device is conveniently provided with a transducer array base. The base of the housing may comprise the plurality of arrays of transducer elements padded with a backing layer and a matching layer. A transducer array base, or the base of the housing otherwise, may include at least one sensor for example selected from the group consisting of temperature sensors, heart rate sensors, cardiovascular sound sensors, bowel sound sensors, sweat analyte sensors, skin stiffness sensors and skin microbial sensors.

Advantageously, the plurality of arrays of transducer elements are arranged, first, to direct ultrasound energy in a predetermined frequency range at an angle of insonation Φ to a subcutaneous region of interest of the subject's body, preferably as instructed by the controller including, or communicable with, processing blocks for the analog front end (AFE), beam former with front end processing, and the backend processing block. The AFE may be implemented in the form of a fully integrated chip single-chip per 2, 4, 8, 16, 32 etc channels or in a multichip per channel solution. The AFE block may be implemented through field programmable gate array (FPGA) or ASIC advantageously implemented using chip(s). An FPGA based controller, which may store and generate pre-programmed digital signals containing ultrasonic oscillation settings is an option.

The beamformer comprises two parts, the transmit beamformer that has the function of initiating scan lines and generating a timed digital pulse string to the transducer elements. The digital pulse string is internally converted into high voltage pulses for the transducer elements so that the transducer elements transmit ultrasound energy in the predetermined frequency range. The receive beamformer has the function of receiving the echo signals from the AFE in the predetermined frequency range and collating the data into representative scan lines through filtering, windowing, summing and demodulation. The two beamformers are time synchronised and continuously communicate timing, position and control data to each other. The receive beamformer is also preferably implemented by FPGA for the portable ultrasound device.

The back-end processing block preferably includes B-mode, Doppler (preferably pulse wave doppler or most preferably continuous wave doppler) and colour flow processing functions and a user of the portable ultrasound device may toggle between these. The B-mode receives demodulated and compressed scan lines and uses interpolation and gray scale mapping to form 2-D gray scale images from the scan lines produced by the receive beamformer. The backend processing block, in this case, preferably produces an image that is ready for use by non-specialist staff in conducting a cannulation. This may involve use of an enhancement technique such as frame smoothing and/or edge detection.

Desirably, the screen forming part of the portable ultrasound device is a colour display unit with non-limiting examples being LCD, LED, OLED, AMOLED or trans-reflective with or without memory in pixel displays. The screen is preferably compatible with touchscreen functionality and is preferably flat. The sub-cutaneous, preferably vascular, structure is conveniently displayed on the screen (which comprises 1280x720 pixels, preferably less than 720x480 pixels, more preferably less than 640x360 pixels and most preferably less than 215x180 pixels) in real time and in a manner not requiring the user of the portable ultrasound device to interpret an ultrasound image alone. This differs from the range of operations conventionally performed by ultrasound operators such as manually adjusting an ultrasound probe's orientation, manually calibrating the operating field of view, setting gain levels, setting contrast levels, setting imaging depth or doppler parameters, all while interpreting an abstract 2D grayscale (or 2D colour-doppler) taken in a single scan line.

The screen, or display unit of which it forms part, has one or more of the following characteristics: preferably lightweight, preferably hydrophobic, preferably oleophobic and preferably chemically resistant to allow for easy sterilisation.

The screen desirably, conveniently with the assistance of the processor, provides an indication of the correct location for insertion of a vascular access device in the form of a cannula or like device. To that end, representation(s) on the screen may display information including one or more of: the depth of an imaged sub-cutaneous structure; and the position of a needle tip being inserted into the tissue. The representation(s) on the screen may indicate one or more of: the calibre of the imaged sub cutaneous structure, for example a vascular structure, and the position of a needle tip being inserted into the tissue. In embodiments of such processing and software indications, algorithms advantageously automatically recognize when a cannula is inserted into a vein and may represent that insertion for both a singular transducer or as the schematic representation of the path segment. For example, in an embodiment involving a schematic vein path representation determined from peak energy signatures

(determined from either FFT or PSD), the original peak may become a trough or, alternatively, a "trough-like" shape such as a square or triangular dip in the energy signature, indicating disruption to the flow-derived energy as a result of the cannula tip disrupting the flow.

In another embodiment in which a B-mode or colour doppler method is used, image enhancement methods may be used to accentuate the intensity and acoustic reflection off the needle tip within both tissue or inside a vein and a bright spot may be represented to display the cannula tip at depth. In this embodiment, the location of the cannula tip within the vein can be used to create a schematic vein path representation, indicating the segment of the vein homing the cannula.

The processor may be programmed with instructions to calculate an optimal needle gauge and/or insertion angle recommended for access to the imaged sub-cutaneous structure, for example a vascular structure, by the vascular access device. The processor may be provided with instructions to select the vascular access device (cannula type) based on selected parameters which may, for example, include the group of vascular diameter, length of vein path and expected flow rates of both vein flow or infusion of fluids such as drugs.

As previously described, path of a vascular structure can be determined through interpolations between a plurality of transducer elements. In an embodiment involving a schematic vein path representation determined from peak energy signatures (determined from either FFT or PSD), vein diameter may be determined based on the features of the characteristic energy-position curves.

Automatic feature recognition methods can be used in embodiments of B-mode or Doppler ultrasound to automatically determine circular or elliptical objects within the field of view and vessel diameter or ellipticity can be calculated automatically by the processor. Based on vessel diameter or ellipticity, cannula size may be determined by the processor and displayed on the screen.

The processor can also be programmed with instructions to determine blood flow rates or velocities in a vascular structure using ultrasound data processed by the processor. Given fluid flow rates through cannulas need to ideally match venous blood flow rates, the processor is conveniently programmed with instructions to select and display a recommended cannula dependent on blood flow rate.

Representations in 3D may be provided by the processor and displayed on the screen for both vascular structure and haemodynamic fields such as, but not limited to one or more of: flow rate, velocity, pressure, shear stress, turbulence, stagnation, pulsatility or stenosis. Such measurements may be represented as time dependent, for example in the form of averaged graphs or real time display of the magnitude of the calculated haemodynamic parameter overlaid on reconstructed 3D structures. Calculated haemodynamic parameters such as shear stress or turbulence can be indicative of disruption of flow due to the inserted vascular access device, for example a cannula. Such parameters may be monitored to facilitate a cannulation process, for example - and without limitation - by determining a recommended time for replacement of the vascular access device and/or to assess the effectiveness of vein flushing with saline to keep the vein open and otherwise manage patency of the vein.

The portable ultrasound device may be used to determine a vascular structure, or vessel, most suited for selected drug infusions. Better flow in larger vessels, for example the cubital fossa of the forearm, is generally more suitable for iron or potassium infusions. Such vessels clear the infused treatments and tend to be less irritated and damaged compared to smaller, superficial vessels with much slower flows. The processor may process vessel structural characteristics and measured haemodynamic parameters to determine the optimal vessel for delivery of a selected infusion.

Mechanical indication for cannulation may also be provided where the portable ultrasound device housing is provided with a base plate with a needle guide to guide the placement of a cannula. The needle guide may include a notch, which is preferably a triangular or rectangular extrusion cut to the front of the base, but most preferably a spherical cut. Preferably, the notch will be less than 3 mm in size, and more preferably less than 1.5 mm in size, in order to restrict movement along the plane of needle insertion.

The screen, or display unit of which it forms part, may be hinged or flexed to move between a display position where it is at an acute angle to the housing and a position in which the screen may be flattened onto the housing of the device, making it possible to reduce the volume consumed while allowing for desirable user ergonomics during usage. This is particularly useful in paramedical, agricultural, military or other on-field applications of the technology where equipment space is constrained. The portable ultrasonic device may be configurable to allow display of the image on another device such as a computer or smartphone screen. However, this is not essential and there is no requirement to require a further device to display the image as a screen is provided as part of the housing of the present portable ultrasound device.

A base of the housing of the portable ultrasound device is conveniently placed in contact with the skin of a patient, the base thus having a contacting area with the skin of the patient. The base preferably has a curved shape, optionally being concave away from the skin surface, allowing convenient placement close to a limb, for example an upper limb such as the forearm of a human subject. The base of the portable ultrasound device may have a surface area of less than 150 cm², preferably less than 60 cm². More preferably, the surface area of the base will be less than 50, 45, 40 or 35 cm², most preferably less than 30 cm² or 25 cm².

The portable ultrasound device is also conveniently lightweight, the mass of the device preferably being no greater than 400g, preferably less than 350g, more preferably less than 200g and most preferably less than 100g or even 75g.

Preferably, the portable ultrasound device includes a memory to store images and other data, such as patient and location data, if required. Preferably, an internal device storage is installed - conveniently as a microSDXC - of less than 512 GB capacity, more preferably less than 256 GB or 128 GB. When full, storage can be easily deleted or transferred to a reference back up drive through means described above. Alternatively, or additionally, to storage onboard the device, storage may be remote with communications between portable ultrasound device and storage device being implemented as described above. Data communicated in this way may be encrypted dependent on the application.

Data obtained from, or from using, the portable ultrasound device may be securely transmitted to a cloud-based, or other, system operated by an organisation utilising it. The system may store electronic medical records or information for insurance purposes. In one advantageous embodiment, the portable ultrasound device may be used in a pathology or blood collection application as results of blood tests can be automatically tracked and updated from the time of collection.

A handheld ultrasonic scanner, conveniently in the form of a cylindrical pipe, may be connected to the housing of the portable ultrasound device, for example through a USB port provided in the housing. The scanner desirably has Doppler functionality (preferably pulse wave doppler or most preferably continuous wave Doppler) and hence the ability to image sub-cutaneous structures, especially vascular structures, when the base of the device is too large for the surface of the skin of an imaged subject, such as - but not limited to - neonates, paediatrics or medium to small animals, including but not limited to dogs, cats, rodents, birds or rabbits. The scanner may also be useful when imaging is required in tight corners, creases or localised areas of physiological interest in the subject. Preferably, the scanner is less than 35 cm³in volume, more preferably less than 25 or 20 cm³in volume, most preferably less than 15 cm³in volume. Preferably, a skin contacting base of the scanner is less than 1 cm²in area, more preferably less than 0.75cm² or 0.6 cm²in area and most preferably less than 0.5 cm²in area. Preferably, at least one of processing, storage and display of images is performed using the housing of the portable ultrasound device which serves as a base unit for the attached hand held scanner.

The portable ultrasound device may include a reading of a tracking device, such as a QR code or barcode. This enables scanning of tracking devices containing relevant data, for example standard electronic patient data including - but not limited to one or a plurality of - patient ID, age, sex, key patient history, location of ultrasound imaging, time of ultrasound imaging and reason for ultrasound imaging. Barcodes, for example, are typically used to store such data in hospitals, clinics, pathology or blood collection centres and other clinical or non-clinical settings.

Conveniently, the housing also includes a power source, such as a rechargeable battery for powering the portable ultrasound device. Connection to other power sources is also possible. The housing may include a DC to DC converter to boost battery voltage to the voltage, say up to 200 volts, to allow the controller to excite transducer elements to transmit ultrasound energy.

In another aspect, the present invention provides a method for non-invasively imaging a selected sub cutaneous structure in a subject, comprising:
continuously transmitting ultrasound energy in a predetermined frequency range to a body of the subject via a plurality of arrays of transducer elements contained in a portable ultrasound device applied at or proximate to a location on the body of the subject, each array of said plurality of arrays of transducer elements being arranged in parallel and each transducer element comprising a transmitter transducer and a receiver transducer, said plurality of parallel array of transducer elements enabling imaging of the sub-cutaneous structure in multiple transverse and lateral planes;
receiving echo signals in a predetermined frequency range from the body of the subject following reflection of ultrasound energy;
processing said received echo signals with a processor; and
producing an interpretable image displaying the sub-cutaneous structure of the subject on a screen (104) forming part of the portable ultrasound device,
wherein said plurality of parallel arrays of transducer elements are operated in continuous wave doppler mode; and
wherein said plurality of arrays of transducer elements are spaced apart from each other by a distance ξ along a horizontal axis selected to minimise interference and maximise scanning window, ξ being between 5 and 30mm.

The portable ultrasound device, as described above, is conveniently brought into contact with the subject and, more particularly a limb such as the forearm of a human patient. Conveniently the user deploys a detachable fastening means to secure the portable ultrasound device in position without needing to manually hold it in position. The detachable fastening means may be a single or multiple use strap, band or belt, preferably made of common medical grade material such as fabric or silicone. Preferably, the fabric material will be compatible with standard sterilization and cleaning mechanisms though may be disposable. A hypo-allergenic non-latex based material would be suitable for a disposable fastening means.

The strap can be in the form of a sleeve, wrap or cuff which attaches to the device and contacts a larger surface area of the limb.

The fastening means desirably allows adjustment of fastening force, for example through an adjustable clasp. The fastening force, similarly to a commonly used tourniquet, is advantageously selected to increase intra vascular pressure and hence engorge the vessel and improve visibility and reduce blood velocity to a range pre-programmed to be extracted by digital processing algorithms.

Following, positioning and optional fastening of the portable ultrasound device proximate the user is assisted to locate an optimised location of the vascular structure, typically a vein, for cannulation. As described above, a base plate of the portable ultrasound device base plate is conveniently provided with a needle guide to guide the placement of a cannula through alignment of the image of the selected vein with the needle guide. The needle guide may include a notch, in which case, alignment is made between the notch and the image of the selected vein. This feature facilitates selection of correct orientation for the portable ultrasound device without guesswork or excessive manual adjustment.

The user can then proceed to inserting a needle or cannula, being assisted by the needle guide and the image displayed on the screen, which act to guide the user to an optimal spot of cannula insertion.

A consumable acoustic conductive patch may be applied to the base of the portable ultrasound device prior to use. Preferably, a double-sided consumable acoustic conductive patch is attached to the base of the portable ultrasound device, conveniently through means of a medically compatible removable glue or a double-sided sticky tape. A sticky affixation end of the consumable patch conveniently attaches to the base of the device. A portion of the consumable patch may be peeled off to expose a medically sterile gel, contained within said patch, which aims to reduce conductance of ultrasonic waves through air, typically the cause of signal noise and signal loss, while maintaining the sterile field needed for venepuncture. Preferably, the volume of the sterile gel in such a preferred patch is less than 120 cm³. More preferably, the volume of the gel in such a preferred patch is less than 110 cm³ or 100 cm³ but most preferably less than 90 cm³.

A consumable conductive patch, preferably including an external sterile gel pocket, may conform with the form factor of the portable ultrasound device, for example being provided in the form of a custom sleeve, envelope or pocket. Such a patch may cover the portable ultrasound device, conveniently being applied thereto through a medically compatible removable glue. The external sterile gel pocket may conveniently be exposed through means of peeling as described above.

The portable ultrasound device and imaging method described above do not require any specialized needles or syringes for cannulation. It allows use of any conventional vascular access devices, such as catheters in any form.

The portable ultrasound device as described is self-contained, conveniently handheld, low cost and simply structured. In contrast to conventional ultrasound equipment, the portable ultrasound device of the present invention can readily be placed into correct orientation for determining a sub-cutaneous structure, for example for cannulation, and does not require complex practitioner adjustments or modular and separate system components consisting of scanning probes, processing units and monitors. The key components, including the transducer elements, processor and circuitry and screen are packaged in a single housing. The portable ultrasound device is conveniently lightweight and may be used to assist cannulation and other ultrasonic imaging of sub-cutaneous structures by a wider range of personnel without formal specialised training including medical and paramedical professionals such as, but not limited to, registered nurses, laboratory phlebotomists, therapists and researchers.

### BRIEF DESCRIPTION OF THE DRAWINGS

Further features of the portable ultrasound device and sub-cutaneous imaging method of the present invention are more fully described in the following description of preferred non-limiting embodiments thereof. This description is included solely for the purposes of exemplifying the present invention. It should not be understood as a restriction on the broad summary, disclosure or description of the invention as set out above. The description will be made with reference to the accompanying drawings in which:
FIG 1 shows a diagrammatic representation of a portable ultrasound device of one embodiment of the present invention and contacted with a forearm of a human patient.
FIG 2 is a block diagram for the portable ultrasound device of FIG 1.
FIG 3a shows a side cross-sectional view of the housing of the portable ultrasound device of FIG 1.
] FIG 3b shows top and side views of the transducer base and arrangement of transmitter and receiver crystal arrays forming transducer element arrays for the portable ultrasound device of FIG.
FIG 4 is a flow diagram of one embodiment of the method for imaging a sub cutaneous structure of the present invention.
FIG 5 shows a schematic representation of the transducer elements in a base of the portable ultrasound device of FIG. 1.
FIG 6 shows the output of the method of FIG. 4 on the integrated display.
FIG 7a is a schematic diagram of the base of the portable ultrasound device of FIG. 1 contacting a conductive material on the skin of a patient.
FIG 7b is a schematic diagram of various components of a single transducer element shown in FIG 7a involved in scanning a vessel.
FIG 8 is a schematic diagram showing transformation of amplitude vs time signals to amplitude vs frequency data by Fast Fourier Transformation (FFT) according to an embodiment of the method of the invention.
FIG 9 is a schematic diagram showing discrimination of arterial and venous structures through an energy signal based algorithm according to an embodiment of the method of the invention.
FIGS 10(a) and (b) are schematic diagrams showing determination of vascular structure depth by an energy signal based algorithm according to an embodiment of the method of the invention.
FIG 11 is a schematic diagram showing vascular structure characterisation through B mode imaging according to an embodiment of the method of the invention.
FIG 12 is a schematic diagram showing vascular structure characterisation through B mode and colour doppler imaging according to an embodiment of the method of the invention.
FIG 13 is a schematic diagram showing estimation of vascular diameter based on an energy signal according to an embodiment of the method of the invention.
FIG 14 is a schematic diagram showing vascular structure characterisation from ultrasound imaging using a compression method according to an embodiment of the invention.

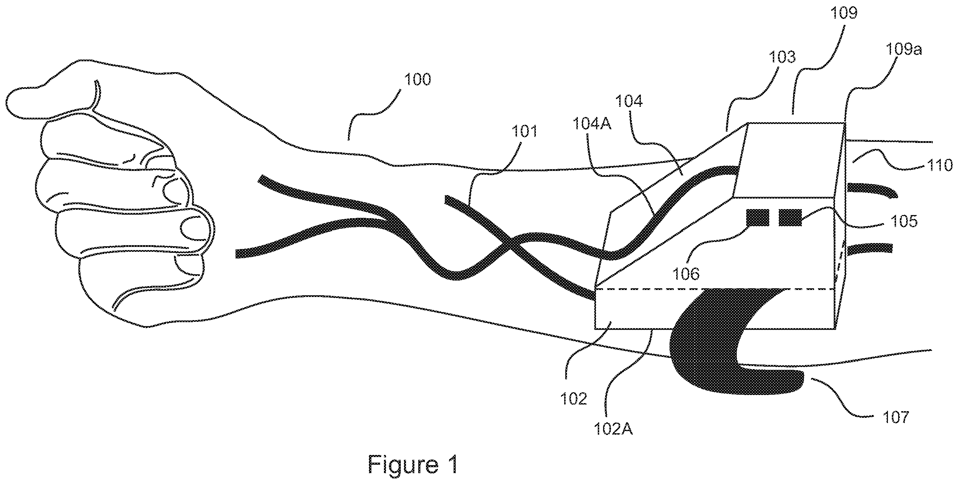

FIG 1 shows a self-contained portable ultrasound device **109** that is used, through continuous transmission and receiving of ultrasound energy, to detect sub-cutaneous structures, here peripheral veins **101** at a depth of 2 to 3 cm below the skin of a patient's forearm **100** with the object of assisting cannulation. A sub-cutaneous structure could be at a lower depth, for example 5 mm, determined by the dead zone of the portable ultrasound device **109.** The portable ultrasound device **109** is self-contained, hand-held, low-cost and simply structured with excessive circuitry required for whole body imaging not necessary for vascular imaging being excluded to reduce size and processing requirements. Portable ultrasound device **109** can be used by personnel without specialist knowledge of ultrasound imaging and who perform most cannulations (typically junior doctors and nurses).

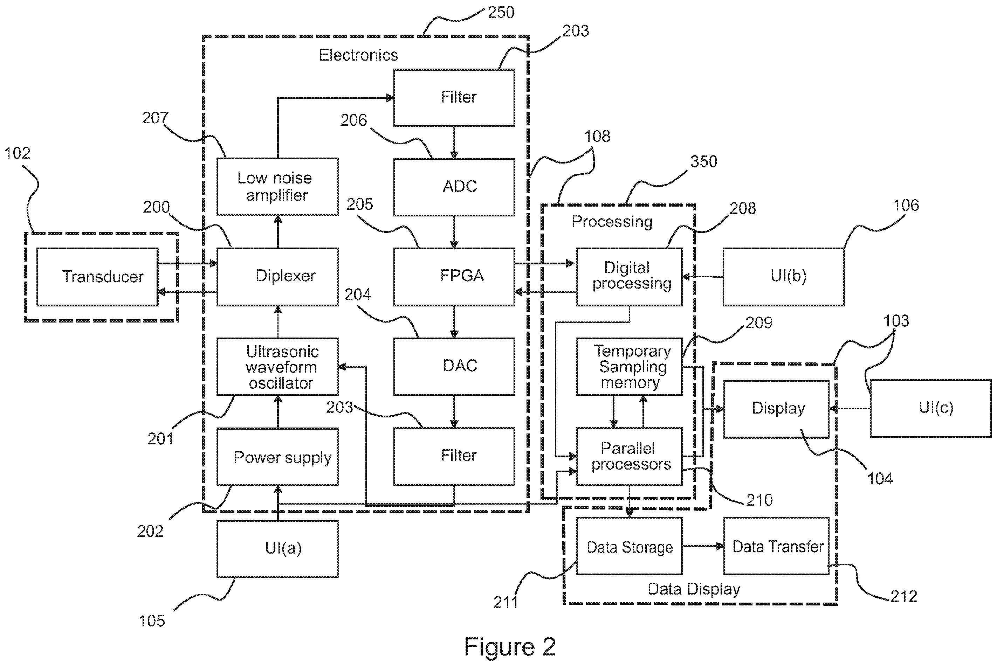

In contrast to conventional complex ultrasound equipment systems, such as those used for whole body imaging, portable ultrasound device **109** does not include multiple user controls or require modular and separate system components consisting of scanning probes, external processing units and monitors together with a requirement for fine adjustments of a number of ultrasound equipment parameters (such as gain) as would be understood for conventional ultrasound imaging equipment in the art of medical ultrasonic imaging; all key components including the ultrasonic transducer elements **102,** controller **250** and processor **350** including processor blocks **208-210,** power (FIG 2: **202)** and an integrated display unit **103,** including a screen **104,** are packaged on-board portable ultrasound device **109** in a single housing **110** which makes its use easier than current options.

Digital processing algorithms simplify traditional B-mode or colour doppler (preferably pulse wave doppler or most preferably continuous wave doppler), ultrasound images which can be abstract to interpret, to provide a two dimensional image of a candidate vein **101** for cannulation on screen **104** which, forming part of housing **108,** is easier to observe than the adjacently disposed screens of prior practice. Rather than reconstructing raw signals (amplitude and frequencies) into grayscale images (B mode) which involves mapping corresponding signal receiving transducer crystal positions, the raw signals (and corresponding positions) are processed simply through frequency domain signal processing and discrimination methods, such as through pulsatility metrics as described above. Pulsatility index, being defined as (systole-diastole) divided by mean blood velocity in the vessel, may be used in this embodiment though other metrics are available as described below.

Frequency domain signal processing and discrimination methods represent less computation for the reconstruction of B mode images and less storage (both temporary and permanent) making hardware requirements simpler. However, the user may choose to visualise and store raw images (B mode) of the subcutaneous structure, using the display mode switch **106** if preferred.

The integrated display unit **103,** screen **104,** power switch **105** and display mode switch **106** represent the exclusive and simple user interfaces for key functions for portable ultrasound device **109.** Due to the configuration of the controller **250** and processor blocks **208-210** of processor **350** for the portable ultrasound device **109,** there is no need for the user to access, additional functions (such as gain) or be provided with functionality to adjust the field of view, for gain adjustment or contrast adjustment. If adjustment of such settings is necessary, an authorised user - such as a maintenance engineer or repairer - could make such adjustments. This would not typically occur, or required to occur, during the cannulation procedure.

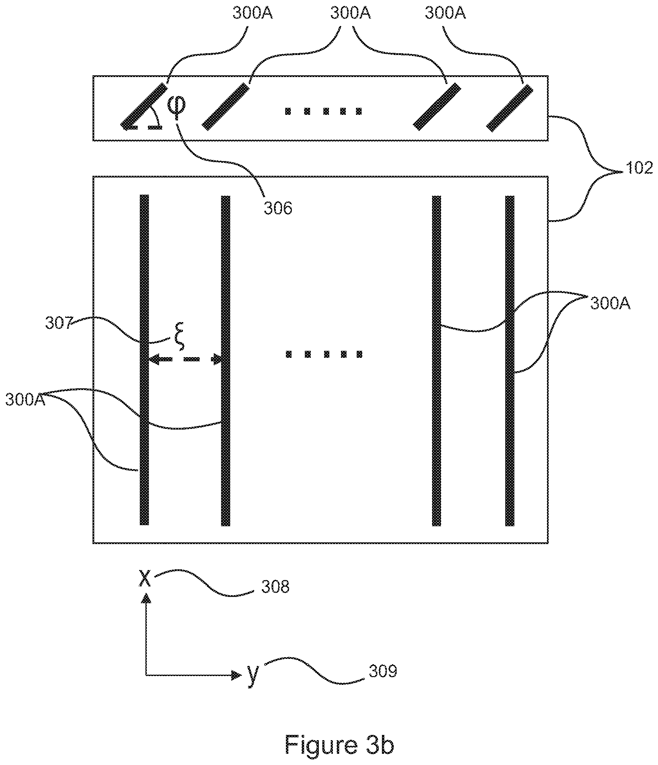

Turning now to FIG 2, FIG 3, FIG 4, FIG 5, FIG 6 and FIG 8, in summary, the portable ultrasound device **109** is - through selection by the controller **250** of transmission signals and processing of received echo signals by processor **350** and its associated processor blocks **208-210** - able to locate peripheral veins **101** in the lateral direction **308, 309** (as shown in FIG 3b) and at various selected depths from 5 mm to 2 to 3 cm, up to 7 to 15 centimetres in exceptional central vessel cases, and through dedicated processors perform signal processing **203, 205, 206** and digital processing block **208** to display a simplified longitudinal representation **104A** of the peripheral vein **101** which can then be shown in real-time on the screen **104** of an integrated display unit **103,** which updates and displays the vein **101** path as the portable ultrasound device **109** is moved over the surface of the skin of the patient's forearm **100.**

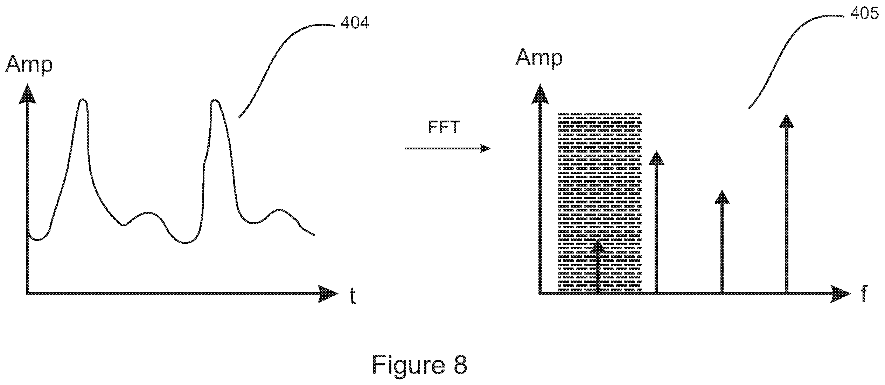

Portable ultrasound device **109** allows discrimination between peripheral veins and arteries. Digital processing block **208** involves execution of an algorithm as embodied in electronic instructions allowing such discrimination through measurement of pulsatility. Veins are typically far less pulsatile than arteries, if pulsatile at all. Pulsatility metrics suitable for measurement and input to such an algorithm here include pulsatility index as described above as determined from velocity waveforms (if determined in the time domain) or high amplitudes at low frequencies (if determined in the frequency domain following Fast Fourier Transform (FFT) as schematically shown in FIG 8). Digital processing block **208** may then prevent any representation of an artery appearing on the screen **104** of integrated display unit **103.**

FIG 2 represents the system block diagram for the portable ultrasound device **109.** Device **109** is provided with functionality for controlling and processing transmission and receival of ultrasonic waves from transducer crystal array **102** and the display or storage of imaged data. Excessive circuitry required for whole body imaging and not specifically necessary for vascular imaging are deliberately excluded from the system to reduce size and processing requirements and to ease use of the portable ultrasound device **109.**

FIG 2 illustrates a Field Programmable Gated Array **205,** within controller block **250** which acts as a microcontroller for portable ultrasound device **109** and stores and generates pre-programmed digital signals containing ultrasonic oscillation settings, which is in turn converted to an analogue signal using a Digital to Analogue Converter (DAC) **204** and filtered for noise **203.** The filtered signal is sent to an ultrasonic waveform oscillator **201** which generates and transmits a primed signal to a diplexer **200** for frequency-domain multiplexing. It will be understood that FPGA **205** can be replaced with a chip-based controller, such as an ASIC chip based controller and there may be other acceptable alternatives.

The ultrasonic waveform oscillator **201** can be powered by a high-voltage power source **202** which can be toggled on or off using the user interface **105.** The array of transducer elements **102** thus transmits and receives ultrasonic signals at the pre-determined frequencies, say in the range 3 to 8 MHz with 7 MHz being selected here (this comparing with the wide frequency range 2 to 18 MHz for typical diagnostic sonographic scanners - see "Application of Ultrasound in Medicine" at www.ncbi.nlm.nih.gov>pmc>articles>PMC3564184 - towards and from the examinee, respectively.

Received signals from transducer crystal array **102** are amplified using a low noise amplifier **207,** filtered **203** and returned to the FPGA microcontroller **205** as a digital signal using an Analogue Digital Converter (ADC) **206.** The received signals are processed onboard the portable ultrasound device **109** by processor block **210** using digital instructions or algorithms described below. As the calculations are complex, parallel processing is implemented at processor block **210.**

Though on-board processing by processor **350** is most preferred, it is possible to reduce processing constraints on the portable ultrasound device **109** by performing processing using a cloud-based system such as that provided by Amazon AWS, whereby data are wirelessly transmitted, processed and returned to the device **109.** In this example, trivial and junk signals are automatically excluded while Doppler frequencies are calculated for non-trivial signals and stored on the temporary sampling memory **209.**

The digital processing block **208** executes instructions, by methods such as those described below, convert the raw signals into a simplified vein representation **104A** which is displayed on screen **104** of the integrated display unit 103.

The display mode switch **106** is toggled to switch between a simplified vein representation **104A** or conventional B-mode or colour-doppler images.

Data related to the ultrasound imaging process for portable ultrasound device **109** are stored on a device memory **211** or transferred to another device **212** by means of reversible USB-C, Bluetooth 5.0, or wireless internet in the form of standard WiFi, dual-band, Wi-Fi Direct or hotspot.

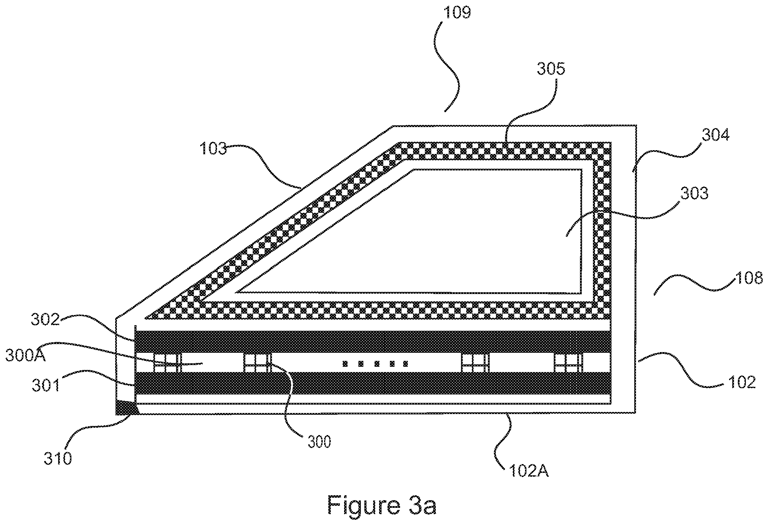

FIGS 3a and 3b show an ultrasonic transducer crystal array **102** located at the base **102A** of the housing **110** of portable ultrasound device **109** for transmitting and sensing reflected waves of the pulse ultrasonic signals. In this case, the base **102A** is comprised of individual transducer elements **300** padded with a backing layer **302** and a matching layer **301.** The transducer elements **300** are such that there is at least one emitter or transmitter crystal **705** and one receiver crystal **707** per transducer element 300 separated by a septum **706** with acoustic insulation properties though other arrangements of transmitter crystal **705** and receiver crystal **707** are possible. Transducer elements **300** may include a piezoelectric material such as PZT, or films, PVDF, PMN-PT, PMN-XX, PIN-PMT-XX where the XX represents the several derivatives of the materials, silicon, metal, CMUT, MEMS, NEMS and so on. The transducer elements **300** are conveniently selected from the group consisting of capacitive micromachined ultrasonic transducers (CMUT), piezoelectric micromachined ultrasonic transducers (PMUT) and similar configurations that use semiconductor technologies to produce the transducer elements **300** of the entire transducer crystal array **102.**

Transducer elements **300** are connected to the electronics package **303** also including the on-board battery. In this example, the electronics package **303** is surrounded by a heat exchange and dissipation mechanism **305** which is in turn surrounded by an acoustic insulation layer **304.**

The portable ultrasound device **109** includes a plurality of linear transducer arrays **300A,** here four arrays, with the transducer arrays **300A** - as shown in FIG 3b - being oriented parallel to each other and carried by the housing **110** of device **109.** The plurality of parallel transducer arrays **300A** provides the ability to image vein **101** in multiple transverse planes to display the lateral position of the vessel on screen **104.**

The parallel transducer arrays **300A** are angled at an angle of insonation Φ where 10 < Φ < 60, as shown in FIG 3b, to ensure the Doppler effect is captured and not nullified. The angled configuration, being fixed, also saves the user from requiring to manually adjust the operating field of view and imaging angles. In this example, parallel transducer arrays **300A** are separated by a distance ξ along the horizontal axis **309,** chosen to minimise interference and maximise the scanning window **708.** ξ may, as described above, be in the range of 5-30mm. In this embodiment, a 15 mm spacing is provided between each parallel array 300. This would cover a region of interest 45mm. A spacing above 30mm would make the portable ultrasound device 109 larger than desirable for use in a cannulation procedure.

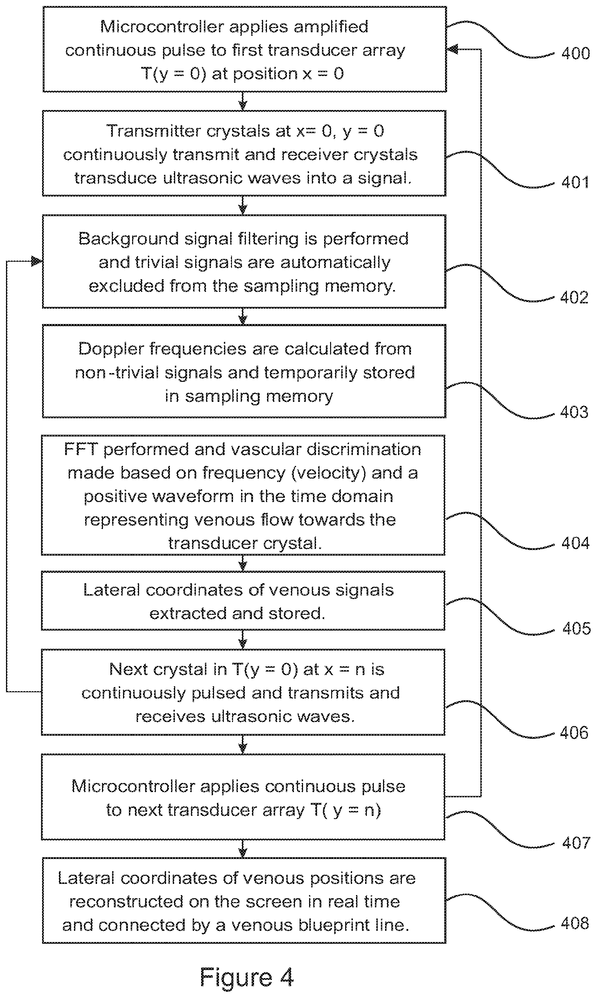

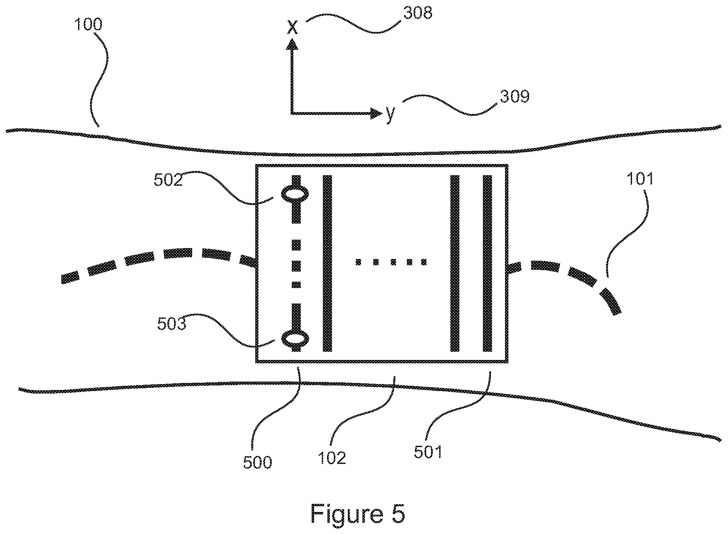

FIG 4 shows a flow diagram for a mode of ultrasonic signal acquisition for use in imaging the vein **101** for cannulation. The FPGA microcontroller **205** applies a continuous pulse throughout operation, thus providing continuous wave operation, starting with emitter crystals **705** of the first transducer array (T(y=0) at position x=0) at the boundary (step **400),** shown in FIG 5 at **503** of the first transducer **500.** Reflected ultrasound waves received by receiver crystals **707** are transduced into a voltametric signal (step **401)** which is then filtered (step **402)** to remove trivial signals from entering sampling memory **403.**

Doppler frequencies and corresponding coordinates are temporarily stored in the sampling memory (step **403)** before a Fast Fourier Transform (FFT) is applied to determine the received frequency and then calculate a frequency shift (Doppler shift). This Doppler shift can help discriminate veins **101** and arteries **101A** based on an energy signal determined from the frequency shift, with positive waveforms **505** representing flow towards the transducer receiving crystals (i.e. blood flow in a vein or vice versa) **707** (step **404).** This characterisation may be termed the venous signature of vein **101** which is stored and/or displayed (step **405)** as venous path **104A** on screen **104.**

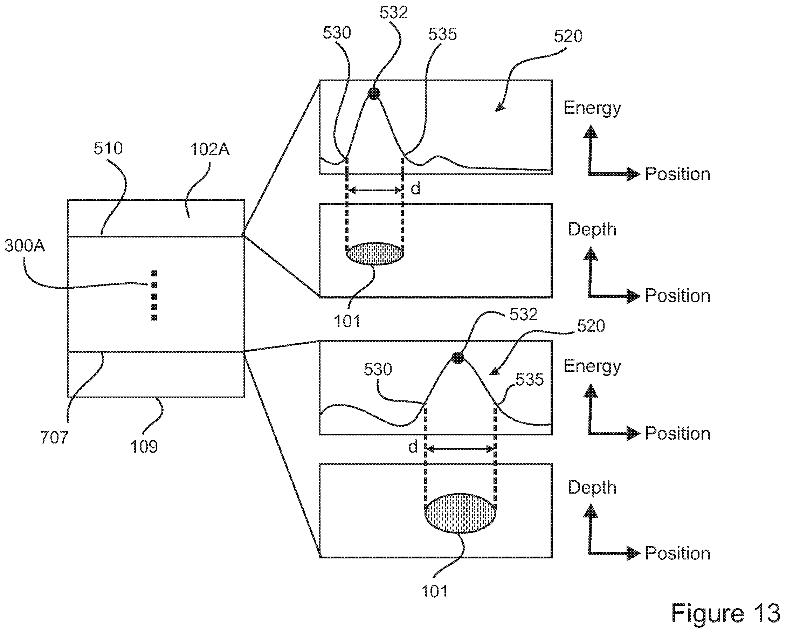

The Doppler shift can also be used to calculate blood flow rates or velocities according to the following formulae: 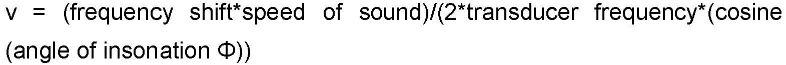 v = (frequency shift*speed of sound)/(2*transducer frequency*(cosine (angle of insonation Φ)) Flowrate = velocity * cross sectional area where cross sectional area is 0.25*pi*diameter² where diameter may, for example, be calculated as described below with reference to FIG 13.

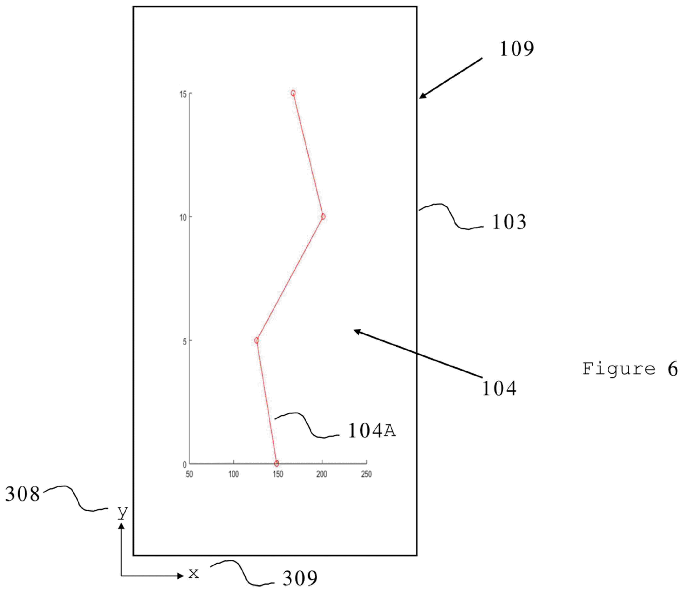

The next transducer crystals **705** are pulsed and the process from steps **402- 406** is repeated until the nth crystal on the first transducer **502,** as shown in FIG 5, is reached. Once the first transducer **500** is cycled, an inter-transducer cycle begins until the nth transducer **501** is reached (step **407).** The lateral coordinates x, y of each venous signature **104A** per transducer element **300, 501, 502** are reconstructed laterally on the screen **104** in real time and connected by a venous blueprint line to form the image **104A** of vein **101** as shown in FIG 6 (step **408).** This image **104A** may be updated continuously on readily viewable screen **104** as the portable ultrasound device **109** is operated by the user.

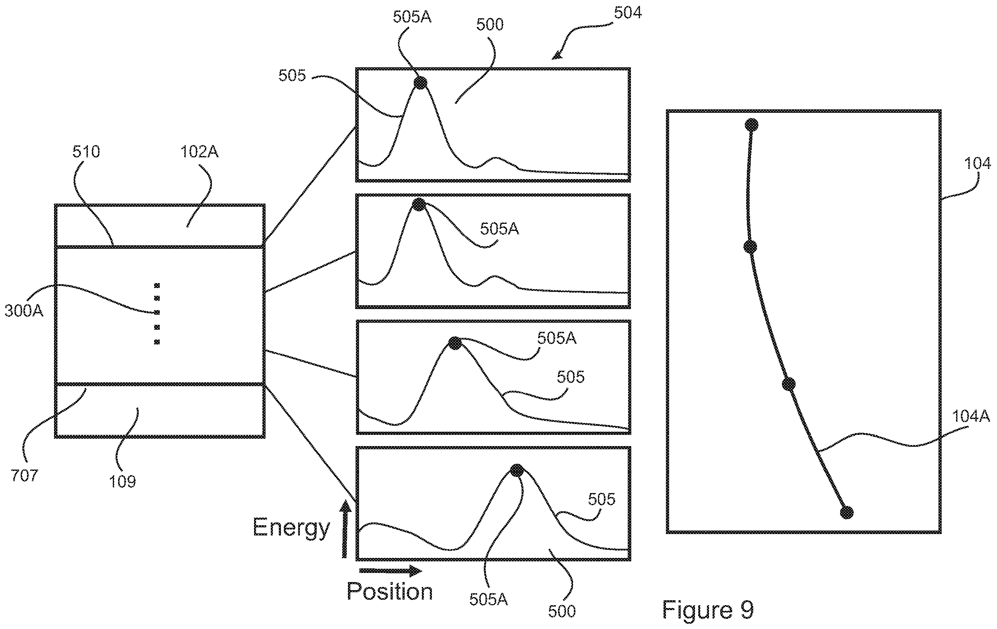

With further reference to step **404,** the processor blocks **208-210** can alternatively be programmed with instructions to discriminate between arterial and venous structures using an energy signature **504** to determine the position of vein **101** below the contacting area of the ultrasound transducers **705, 707** with the patient's skin. The energy signature **504,** as schematically indicated in FIG 9, may be determined from a frequency-domain representation **405** of the raw signal, determined by a Fast Fourier Transform (FFT) of the continuously acquired current signal **404,** as indicated by FIG 8, or more preferably from a power spectral density (PSD) computed from the sampled signal. This energy signature is preferably the amplitude (Amp) of the dominant signal frequency, or alternatively the sum of amplitudes of dominant frequencies or else the sum of amplitudes squared. More preferably the energy is the area under the FFT or PSD distribution. Alternatively, the energy could be the ratio of amplitude to area under the distribution or the ratio of sum of amplitudes (or sum of amplitudes squared) to the area under the distribution.

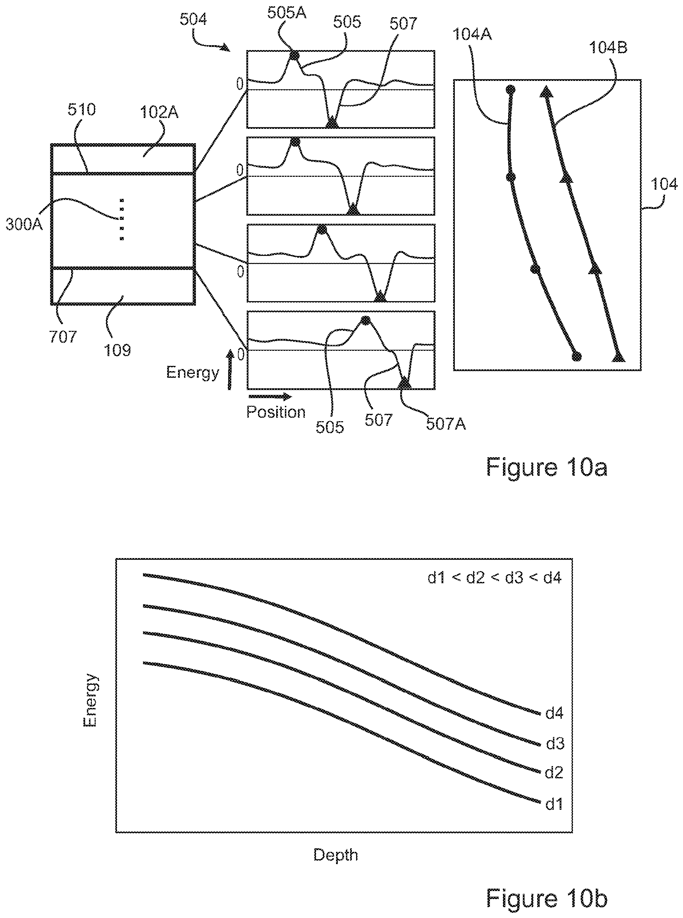

Further referring to FIG 9, and as above described, the positive waveform **505** obtained for each of receiver crystals **510** and **707** and receiver crystals within transducer element array **300A** is representative of a point along a vein **101,** these points having a position which can stay the same or vary as shown in the energy-position trace or waveforms **504** and, as shown in FIG 10 for artery **101A,** waveform **507.** Point **505A** or the peak of the waveform **505** approximately represents the centre of the vein **101** at a given point with maximum energy typically being at the centre of a healthy vascular structure. A negative waveform **507,** as shown in FIG 10, would be representative of a point along an artery **101A** with point **507A** or the peak of the waveform **507** approximately representing the centre of the artery **101A** at a given point with maximum energy typically being at the centre of a healthy vascular structure. The processor **350** displays the points on screen **104** with the result showing the path of vein **101** as line **104A.**

The processor **350** may allow discrimination between a vein **101** and an artery **101A** using the difference between positive (vein **101)** and negative (artery **101A)** waveforms **505, 507.** Vein path is shown as line **104A** and artery path is shown as line **104B.**

The processor **350** also estimate depth of vein **101** below the skin by executing the above described energy signature based instructions and as also schematically indicated by FIG 10. For a vessel diameter, d1-d4, calculated using methods described below, characteristic curves may be inferred relating magnitude of energy to the vascular structure or vein **101** depth for different vessel diameters d1 to d4 with d1<d2<d3<d4 as shown in FIG 10(b) with larger vessels having a higher magnitude energy signature. This depth can be determined, and displayed on screen **104,** as a value in millimetres or centimetres below the skin, or through colour weightings by depth magnitude or through other visual representations such as 3D perception of shallowness or depth. The processor **350** thus allows calculation of depth of vein **101** or artery **101A** beneath the skin without the need for B-mode cross sectional image reconstructions.

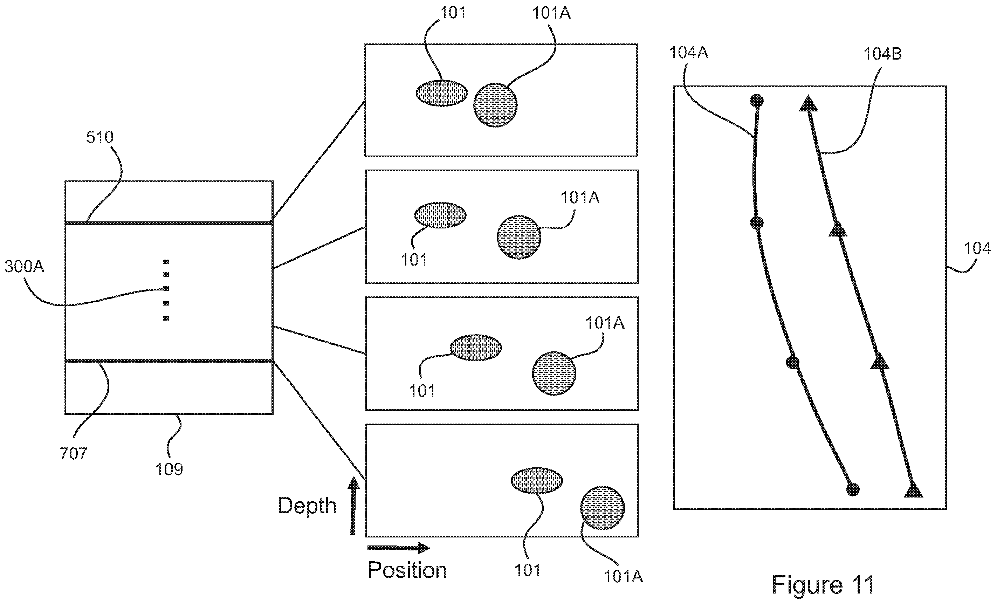

Alternatively to the approaches described above, B-mode imaging may be performed for each transducer **510, 707** within the array as schematically shown in FIG 11. Structural metrics (such as diameter, compressibility (as indicated), and other curve features) can be calculated by processor **350** and used to discriminate between arteries **101A** and veins **101** with paths of vein **101** and artery **101A** being displayed as respective lines **104A, 104B** on screen **104.** To this end, machine learning techniques, for example including the use of convolutional neural networks, can be used to classify and identify veins and arteries, following training datasets produced by a population of experienced ultrasound users. In B-mode or colour Doppler representations, depth of vascular structures can be directly measured and displayed on screen **104** of ultrasound device **109.**

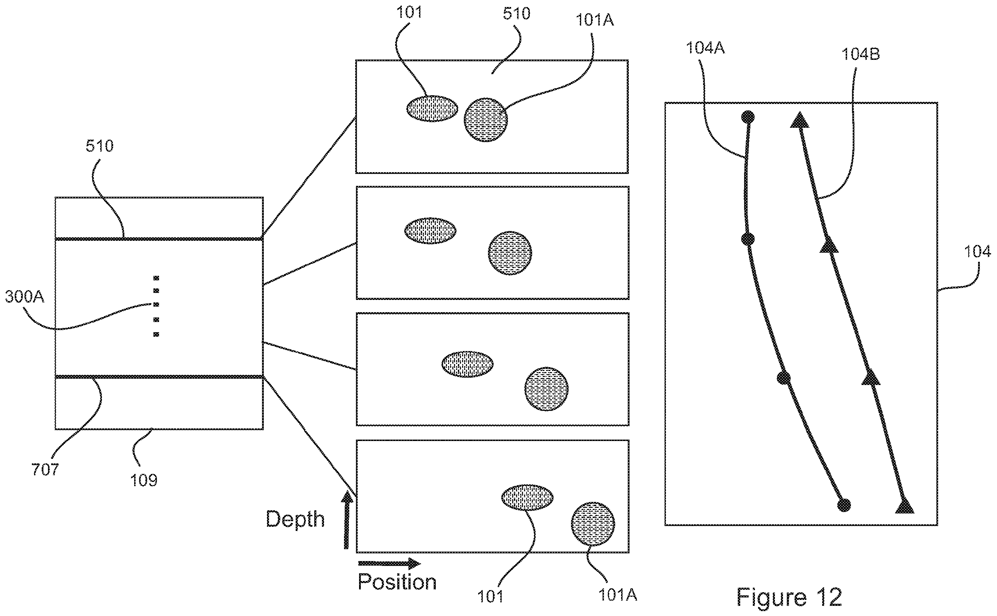

In another embodiment of an algorithm for artery or vein discrimination, as schematically shown in FIG 12, B-mode and Colour Doppler images **510** are acquired and automatic computer vision techniques are used to identify flow away or towards the transducer **510, 707** (or flow away from the heart, representing artery **101A,** and flow towards the heart, representing vein **101).** Colour Doppler represents flow towards the transducer (veins typically) as shades of blue **515** with intensities representing velocity magnitudes, and flow away from the transducer (arteries typically) as shades of red 520. As such, artery **101A** and vein **101** can be easily distinguished through automatic computer vision techniques discriminating colour. In B-mode or colour Doppler representations, path and depth of vessels can be directly measured and displayed on screen **104** of ultrasound device **109** as vein path **104A** and artery path **104B.**

Conveniently, the processor **350** recognizes - in the energy signal based algorithms as described with reference to FIGS 9, 10 and 13 - that the peak in the energy signature forms part of parabolic or gaussian resembling distributions and that the start and end points **530, 535** of such distributions can be used to estimate vein **101** diameter, d, particularly in the lateral direction. A method of determining start or end point **530, 535** - as indicated in FIG 13 - involves calculation, by processor **350,** of gradient at every point along the energy curves **520** and computed by taking the ratio of energy change between consecutive data points to the change in position. A start point **530** may be identified, for example, when a gradient quickly increases from low values (say between - 0.25 to 0.25) to a large value (say between 0.75 to 1). Conversely, an end point **535** may be identified as a change in gradient from a large negative value (say between -0.75 to - 1) to a low value (say between - 0.25 to 0.25). Compared to structural analysis of B-mode cross-sectional images which requires either manual measurements of diameter or automated feature recognition (through artificial intelligence constraints or machine learning methods) for diameter measurements, the energy curve method as described above allows for a more efficient and more objective method to determine diameter. Additionally, no training datasets (labelled) are needed for the automatic diameter calculation, however training machine learning models to optimize the identification of start and end points **530, 535** is possible.

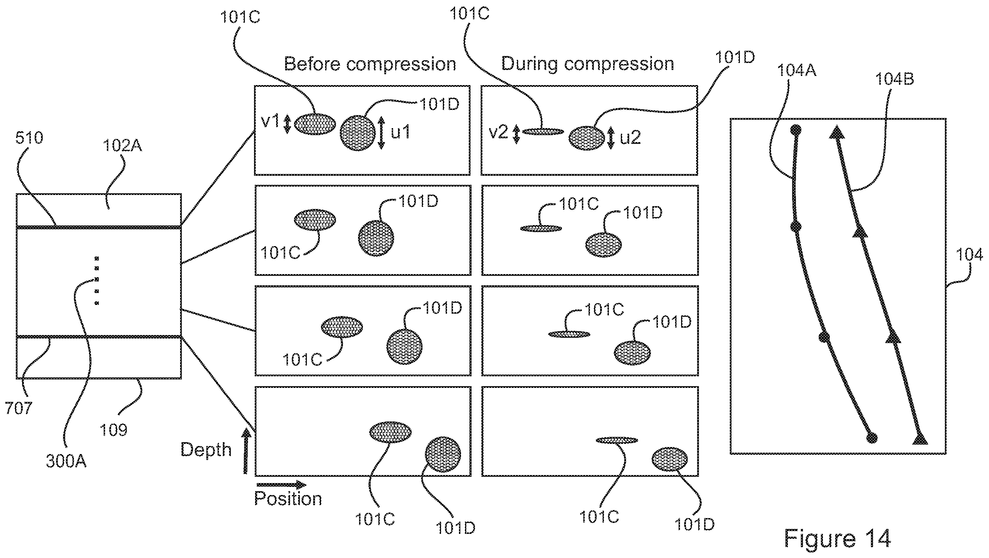

In another embodiment of the method of artery or vein discrimination, ultrasound data is acquired by ultrasound device **109** without and with mechanical compression of the subcutaneous vessels through application of external force by the housing onto the skin. This method is indicated in FIG 14. Taking advantage of different vascular wall properties of veins (compressible but not muscular and not elastic) and arteries (muscular, less compressible than veins and elastic), changes during compression can be measured to distinguish between arteries and veins.

In one embodiment of the compression method, peak energy signatures (determined from either FFT or PSD) change with compression as schematically shown in FIG 14. Notably, the positive peaks 101C (representing a flow in a vein **101** when flowing towards the receiver crystals 510, 707 and those in transducer array 300A) is flattened with compression while the negative peaks 101D (representing a flow in an artery **101A** when flowing away from the transducer), only shows reductions in magnitude while maintaining its directionality (negative peak representing flow away from the transducer). The characteristic drop in the peak 101C (toward zero) for the vein and a linear decrease in magnitude for the artery 101D are preferably calculated through absolute value measurements or more preferably through feature curve analysis to discriminate between arteries and veins. Alternatively, as vascular structure diameter, d, can be determined from energy waveforms **504** as previously described, the change in vascular structure size due to compression can be estimated by calculating the change in diameter from before compression to after compression, i.e. - from u1 to u2 and from v1 to v2. A large deformation (say equivalent to 90 - 100% of the original diameter) is expected for veins compared to arteries. This method also results in vein path **104A** and artery path **104B** being displayed on screen **104** of ultrasound device **109.**

In another embodiment of the compression method utilizing B-mode or colour doppler mode, automatic feature edge recognition is used to identify circular or elliptical objects and the maximum vertical chord length of said objects can be measured. Circular or elliptical objects can respectively be identified as arteries or veins as the reduction in maximum vertical chord length is largest for veins (say equivalent to 90 - 100% of the original diameter) compared to arteries.

In another embodiment of both compression methods described, the compressibility of the vein either through energy drops or chord length drops can be used to indicate the structural integrity of the vein **101** with larger resistance to compression indicating higher stiffness (structural integrity). Additionally, the restoration of structure following compression can be used to determine the extent of plastic deformations and therefore can be used to determine elasticity. The choice of structurally stable veins through both stiffness and elasticity inferences is a critical factor for cannula insertions as well as cannula success throughout in-dwelling periods (no dislodgement, extravasation).

Automatic feature recognition methods can be used in embodiments of B-mode or Doppler ultrasound, as described above, to automatically determine circular or elliptical objects within the field of view and vessel diameter or ellipticity can be calculated automatically by the processor. Based on vessel diameter or ellipticity, cannula size may be determined by the processor and displayed on the screen. For example, in adults, for vein diameters greater than 1.3 mm, the algorithm would most preferably recommend green cannulas (18G; 1.3mm diameter), pink cannulas (20G; 1.1mm diameter) or blue cannulas (22G; 0.9mm diameter). Orange (14G; 2.1mm diameter) or gray (16G; 1.8mm diameter) may also be recommended for larger veins (above 1.8mm), however these are recommended in trauma, resuscitation, rapid blood transfusions, rapid fluid replacement, or surgery, all at very high infusion flow rates (240 mL/min for orange and 180 mL/min for gray).

Calculated haemodynamic parameters such as shear stress or turbulence can be indicative of disruption of flow due to the inserted vascular access device, for example a cannula. Such parameters may be monitored to facilitate a cannulation process, for example - and without limitation - by determining a recommended time for replacement of the vascular access device and/or to assess the effectiveness of vein flushing with saline to keep the vein **101** open. For example, the processor **350** can calculate and represent blood flow rates on screen **104** and use this information to manage a cannulation. Procedure, for example, at a saline flow of 10 mL/h, blood stasis can be indicated on screen **104,** which may occur around the cannula tip as a recirculation (stasis) zone is formed, hence blocking the tip and reducing device patency. Preferably, at 20 mL/h reduction in stasis is indicated and a score for vein patency may be calculated. Most preferably, 30 - 40 mL/h saline is indicated as being most effective for a larger range of venous flow rates and peripheral vein sizes. However, based on patient needs, determined by factors such as hydration, difficulty of access or bruising, larger flow loads above 40 mL/h can be indicated on screen **104** of ultrasound device **109.**

Use of portable ultrasound device **109** is described below.

As the user moves the portable ultrasound device **109** over the skin of the patient's forearm **100** to find a vein pathway (which will be determined, through use of the device **109,** as vein **101),** the user aims to align the simplified longitudinal representation **104A** of the vein **101,** obtained (preferably with other procedural data or recommendations as above described) with the notch on the base **102A.**

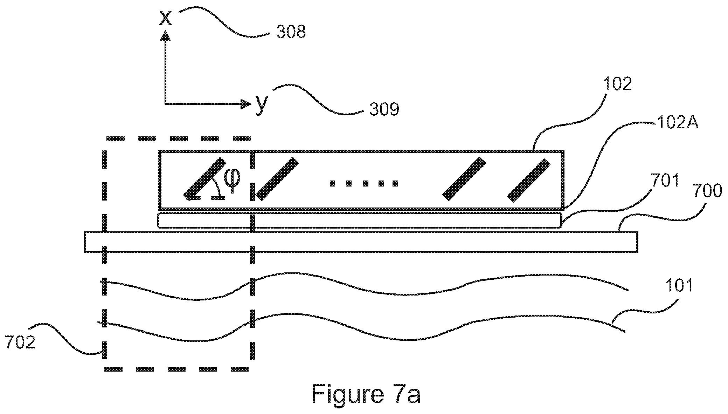

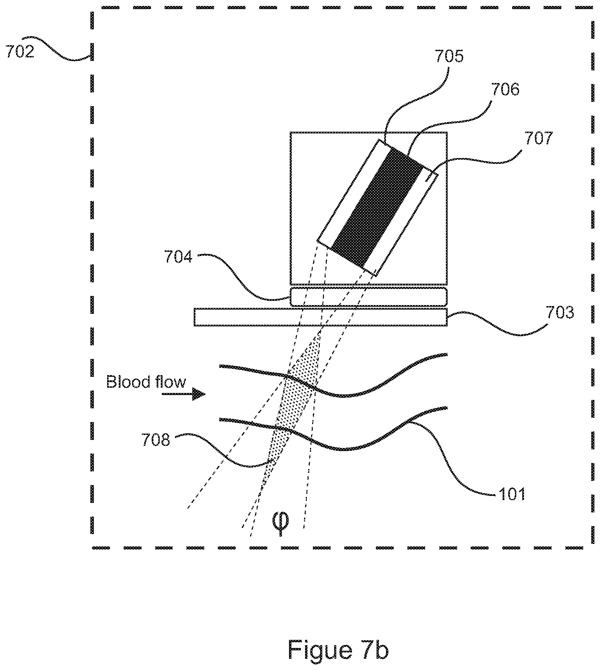

FIG 7 shows an example where the user attaches a double-sided consumable acoustic conductive patch **701** to the base **102A** of device **109,** preferably through means of a medically compatible removable glue or a double-sided sticky tape. The sticky affixation end of the acoustic conductive patch **701** attaches to the base **102A** of the portable ultrasound device **109.** The opposite end of the acoustic conductive patch **701** can be peeled off to expose a medically sterile gel, contained within the patch **701,** which aims to reduce conductance of ultrasonic waves through air, typically the cause of signal noise and signal loss, while maintaining the sterile field needed for cannulation.

Referring again to FIG 1, once aligned, the user deploys a detachable fastening means **107** to secure the portable ultrasound device **109** in position on the patient's forearm **100.** The fastening means **107** can be a single or multiple use strap or band or belt made of common medical grade material such as fabric or silicone, with an adjustable clasp to adjust for fastening force. The fastening force can be likened to that produced of a commonly used tourniquet, required to increase intra vascular pressure and hence engorge the vessel and improve visibility and reduce blood velocity to a range pre-programmed to be extracted by digital processing algorithms **208.**

The user can then conveniently proceed to inserting a needle or cannula through an insertion notch **310** at the base **102A** of the portable ultrasound device **109,** without needing to hold it in place. Hence, the simplified longitudinal representation **104A** of peripheral vein **101** - produced by processor **350** and, in particular, processor blocks **208-210** as described above - guides the operator to the optimal spot of needle insertion with respect to the imaged pathway **104A** of the peripheral vein **101** with benefits for both user and patient.

The portable ultrasound device **109** as described is self-contained, conveniently hand-held, low cost and simply structured. In contrast to conventional ultrasound equipment, the portable ultrasound device of the present invention does not require modular and separate system components consisting of scanning probes, processing units and monitors. The key components, including the transducer elements, processor and circuitry and screen are packaged in a single housing. The portable ultrasound device **109** is conveniently lightweight and may be used to assist venepuncture or cannulation and other ultrasonic imaging of sub cutaneous structures by a wider range of personnel without formal specialised training including medical and paramedical professionals such as, but not limited to, registered nurses, laboratory phlebotomists, therapists and researchers.

## Claims

1. A portable ultrasound device (109) for non-invasively imaging a selected sub-cutaneous structure (101) in a subject, comprising:
(a) a housing (110);
(b) a plurality of arrays (300A) of transducer elements (300), each array (300A) being arranged in parallel and each transducer element (300) comprising a transmitter transducer (705) and a receiver transducer (707), located within said housing (110) for continuously transmitting ultrasound energy in a predetermined frequency range toward a body (100) of a subject and continuously receiving echo signals in a predetermined frequency range from the body (100) of the subject following reflection of ultrasound energy, said plurality of parallel arrays (300A) of transducer elements (300) enabling imaging of the sub-cutaneous structure (101) in multiple transverse and lateral planes;
(c) a controller (250) for operating said plurality of parallel arrays (300A) of transducer elements (300) and communicable with a processor (350) for processing said received echo signals from said plurality of parallel arrays (300A) of transducer elements (300); and
(d) a screen (104) forming part of said housing (110) for displaying an interpretable image (104A) of said sub-cutaneous structure (101) wherein said processor (350) is configured to process said echo signals reflected from the sub-cutaneous structure (101) to selectively produce said interpretable image (104A) of the sub-cutaneous structure (101) of the subject,
wherein the controller (350) is configured to operate said plurality of parallel arrays (300A) of transducer elements (300) in continuous wave doppler mode; and
wherein said plurality of parallel arrays (300A) of transducer elements (300) are spaced apart from each other by a distance ξ along a horizontal axis (309) selected to minimise interference and maximise scanning window (708), wherein ξ is between 5 and 30mm.

2. The ultrasound device (109) of claim 1, wherein said sub-cutaneous structure (101) is a vascular structure, optionally a vein.

3. The ultrasound device (109) of claim 1 or 2, wherein said plurality of parallel arrays (300A) of transducer elements (300) are angled at an angle of insonation Φ where 10<Φ<60.

4. The ultrasound device (109) of any one of the preceding claims, wherein said screen (104) and processor (350) provide an indication of a correct location for insertion of a vascular access device, representation(s) on the screen (104) optionally displaying information including one or more of: depth (d1-d4) of the imaged sub-cutaneous structure (101) and position of a needle tip being inserted into the body of the subject.

5. The ultrasound device (109) of claim 4, wherein representations in 3D are provided for both a vascular structure (101) and haemodynamic fields, optionally one or more of: velocity, pressure, shear stress, turbulence, stagnation, pulsatility or stenosis.

6. The ultrasound device (109) of any one of the preceding claims, wherein said processor (350) is programmed with instructions to discriminate between arterial and venous vascular structures (101,101A) by a time based algorithm based on measurement of pulsatility; or due to intrinsic differences in a measured Doppler shift.

7. The ultrasound device (109) of any one of claims 1-5, wherein said processor is programmed with instructions to discriminate between arterial and venous vascular structures, wherein said processor determines the position of the sub-cutaneous structure below a contacting area of the ultrasound device with the body of the subject based on the processing of an ultrasound energy signal of the received echo signals, wherein the processor determines at least one of the depth and dimension of the sub-cutaneous structure below said contacting area based on processing of the ultrasound energy signal .

8. The ultrasound device (109) of claim 4, wherein the processor (350) calculates at least one of an optimal needle gauge and insertion angle recommended for access to the imaged sub-cutaneous vascular structure (101) by the vascular access device.

9. A method for non-invasively imaging a selected sub cutaneous structure (101) in a subject, comprising:
continuously transmitting ultrasound energy in a predetermined frequency range to a body of the subject via a plurality of arrays (300A) of transducer elements (300) contained in a portable ultrasound device (109) applied at or proximate to a location on the body (100) of the subject, each array (300A) of said plurality of arrays (300A) of transducer elements (300) being arranged in parallel and each transducer element (300) comprising a transmitter transducer (705) and a receiver transducer (707), said plurality of parallel arrays (300A) of transducer elements (300) enabling imaging of the sub-cutaneous structure (101) in multiple transverse and lateral planes;
receiving echo signals in a predetermined frequency range from the body (100) of the subject following reflection of ultrasound energy;
processing said received echo signals with a processor (350); and
producing an interpretable image (104A) displaying the sub-cutaneous structure (101) of the subject on a screen (104) forming part of the portable ultrasound device (109),
wherein said plurality of parallel arrays (300A) of transducer elements (300) are operated in continuous wave doppler mode; and
wherein said plurality of arrays (300A) of transducer elements (300) are spaced apart from each other by a distance ξ along a horizontal axis (309) selected to minimise interference and maximise scanning window (708), ξ being between 5 and 30mm.

10. The imaging method of claim 9, wherein said processor (350) discriminates between arterial and venous vascular structures (101,101A) by a time based algorithm based on measurement of pulsatility; or due to intrinsic differences in a measured Doppler shift.

11. The imaging method of claim 9, wherein said processor (350) discriminates between arterial and venous structures (101,101A) using an energy signature of the received echo signals to determine the position of a vein (101) below a contacting area (102A) of the ultrasound device (109) with the body (100) of the subject.

12. The imaging method of claim 9 wherein said processor (350) discriminates between arterial and venous structures (101,101A).

13. The imaging method of claim 12, wherein said processor (350) determines, the position of the sub-cutaneous structure below a contacting area of the ultrasound device with the body of the subject based on the processing of an ultrasound energy signal of the received echo signals, wherein the processor determines at least one of the depth and dimension of the sub-cutaneous structure below said contacting area based on processing of the ultrasound energy signal.

14. The imaging method of claim 13, wherein the processor (350) processes the ultrasound signal with compression of the sub-cutaneous structure (101C,101D).

15. The imaging method of any one of claims 9 to 14, wherein the processor (350) calculates at least one of an optimal needle gauge and an insertion angle for access to an imaged sub-cutaneous vascular structure (101) by a vascular access device.

## Patentansprüche

1. Tragbare Ultraschallvorrichtung (109) zur nichtinvasiven Bildgebung einer ausgewählten subkutanen Struktur (101) bei einem Subjekt, umfassend:
(a) ein Gehäuse (110);
(b) eine Mehrzahl von Arrays (300A) von Wandlerelementen (300), wobei jedes Array (300A) parallel angeordnet ist und jedes Wandlerelement (300) einen Sendewandler (705) und einen Empfangswandler (707) umfasst, in dem Gehäuse (110) befindlich, um kontinuierlich Ultraschallenergie in einem vorbestimmten Frequenzbereich zu einem Körper (100) eines Subjekts auszusenden und nach Reflexion von Ultraschallenergie kontinuierlich Echosignale in einem vorbestimmten Frequenzbereich von dem Körper (100) des Subjekts zu empfangen, wobei die Mehrzahl paralleler Arrays (300A) von Wandlerelementen (300) eine Bildgebung der subkutanen Struktur (101) in mehreren transversalen und lateralen Ebenen ermöglicht;
(c) eine Steuerung (250), die zum Betreiben der Mehrzahl paralleler Arrays (300A) von Wandlerelementen (300) dient und mit einem Prozessor (350) zum Verarbeiten der empfangenen Echosignale von der Mehrzahl paralleler Arrays (300A) von Wandlerelementen (300) kommunizieren kann; und
(d) einen Bildschirm (104), der einen Teil des Gehäuses (110) bildet, zum Anzeigen eines interpretierbaren Bildes (104A) der subkutanen Struktur (101), wobei der Prozessor (350) dazu ausgebildet ist, die von der subkutanen Struktur (101) reflektierten Echosignale zu verarbeiten, um selektiv das interpretierbare Bild (104A) der subkutanen Struktur (101) des Subjekts zu erzeugen,
wobei die Steuerung (350) dazu ausgebildet ist, die Mehrzahl paralleler Arrays (300A) von Wandlerelementen (300) im Continuous-Wave-Doppler-Modus zu betreiben; und
wobei die Mehrzahl paralleler Arrays (300A) von Wandlerelementen (300) durch einen Abstand ξ entlang einer horizontalen Achse (309) voneinander beabstandet ist, der so gewählt ist, dass Interferenzen minimiert und ein Abtastfenster (708) maximiert werden, wobei ξ zwischen 5 und 30 mm beträgt.

2. Ultraschallvorrichtung (109) nach Anspruch 1, wobei die subkutane Struktur (101) eine Gefäßstruktur, optional eine Vene, ist.

3. Ultraschallvorrichtung (109) nach Anspruch 1 oder 2, wobei die Mehrzahl paralleler Arrays (300A) von Wandlerelementen (300) unter einem Beschallungswinkel Φ angewinkelt ist, wobei 10<Φ<60.

4. Ultraschallvorrichtung (109) nach einem der vorhergehenden Ansprüche, wobei der Bildschirm (104) und der Prozessor (350) eine Anzeige einer korrekten Stelle für das Einführen einer Gefäßzugangsvorrichtung bereitstellen, wobei Darstellung(en) auf dem Bildschirm (104) optional Informationen anzeigen, die eines oder mehrere der Folgenden umfassen: Tiefe (d1-d4) der abgebildeten subkutanen Struktur (101) und Position einer Nadelspitze, die in den Körper des Subjekts eingeführt wird.

5. Ultraschallvorrichtung (109) nach Anspruch 4, wobei Darstellungen in 3D sowohl für eine Gefäßstruktur (101) als auch für hämodynamische Felder bereitgestellt werden, optional eines oder mehrere der Folgenden: Geschwindigkeit, Druck, Schubspannung, Turbulenz, Stagnation, Pulsatilität oder Stenose.

6. Ultraschallvorrichtung (109) nach einem der vorhergehenden Ansprüche, wobei der Prozessor (350) mit Anweisungen programmiert ist, um zwischen arteriellen und venösen Gefäßstrukturen (101,101A) durch einen zeitbasierten Algorithmus auf der Grundlage einer Messung der Pulsatilität zu unterscheiden; oder aufgrund intrinsischer Unterschiede in einer gemessenen Dopplerverschiebung.

7. Ultraschallvorrichtung (109) nach einem der Ansprüche 1-5, wobei der Prozessor mit Anweisungen programmiert ist, um zwischen arteriellen und venösen Gefäßstrukturen zu unterscheiden, wobei der Prozessor die Position der subkutanen Struktur unterhalb einer Kontaktfläche der Ultraschallvorrichtung mit dem Körper des Subjekts auf der Grundlage der Verarbeitung eines Ultraschallenergiesignals der empfangenen Echosignale bestimmt, wobei der Prozessor mindestens eines von der Tiefe und der Abmessung der subkutanen Struktur unterhalb der Kontaktfläche auf der Grundlage einer Verarbeitung des Ultraschallenergiesignals bestimmt.

8. Ultraschallvorrichtung (109) nach Anspruch 4, wobei der Prozessor (350) mindestens eines von einer optimalen Nadelstärke und einem Einführwinkel berechnet, die für einen Zugang zu der abgebildeten subkutanen Gefäßstruktur (101) durch die Gefäßzugangsvorrichtung empfohlen werden.

9. Verfahren zur nichtinvasiven Bildgebung einer ausgewählten subkutanen Struktur (101) bei einem Subjekt, umfassend: Kontinuierliches Aussenden von Ultraschallenergie in einem vorbestimmten Frequenzbereich zu einem Körper des Subjekts über eine Mehrzahl von Arrays (300A) von Wandlerelementen (300), die in einer tragbaren Ultraschallvorrichtung (109) enthalten sind, die an oder in der Nähe einer Stelle an dem Körper (100) des Subjekts angelegt ist, wobei jedes Array (300A) der Mehrzahl von Arrays (300A) von Wandlerelementen (300) parallel angeordnet ist und jedes Wandlerelement (300) einen Sendewandler (705) und einen Empfangswandler (707) umfasst, wobei die Mehrzahl paralleler Arrays (300A) von Wandlerelementen (300) eine Bildgebung der subkutanen Struktur (101) in mehreren transversalen und lateralen Ebenen ermöglicht;
Empfangen von Echosignalen in einem vorbestimmten Frequenzbereich von dem Körper (100) des Subjekts nach Reflexion von Ultraschallenergie;
Verarbeiten der empfangenen Echosignale mit einem Prozessor (350); und
Erzeugen eines interpretierbaren Bildes (104A), das die subkutane Struktur (101) des Subjekts auf einem Bildschirm (104) anzeigt, der einen Teil der tragbaren Ultraschallvorrichtung (109) bildet,
wobei die Mehrzahl paralleler Arrays (300A) von Wandlerelementen (300) im Continuous-Wave-Doppler-Modus betrieben wird; und wobei die Mehrzahl von Arrays (300A) von Wandlerelementen (300) durch einen Abstand ξ entlang einer horizontalen Achse (309) voneinander beabstandet ist, der so gewählt ist, dass Interferenzen minimiert und ein Abtastfenster (708) maximiert werden, wobei ξ zwischen 5 und 30 mm beträgt.

10. Bildgebungsverfahren nach Anspruch 9, wobei der Prozessor (350) zwischen arteriellen und venösen Gefäßstrukturen (101,101A) durch einen zeitbasierten Algorithmus auf der Grundlage einer Messung der Pulsatilität unterscheidet; oder aufgrund intrinsischer Unterschiede in einer gemessenen Dopplerverschiebung.

11. Bildgebungsverfahren nach Anspruch 9, wobei der Prozessor (350) zwischen arteriellen und venösen Strukturen (101,101A) unter Verwendung einer Energiesignatur der empfangenen Echosignale unterscheidet, um die Position einer Vene (101) unterhalb einer Kontaktfläche (102A) der Ultraschallvorrichtung (109) mit dem Körper (100) des Subjekts zu bestimmen.

12. Bildgebungsverfahren nach Anspruch 9, wobei der Prozessor (350) zwischen arteriellen und venösen Strukturen (101,101A) unterscheidet.

13. Bildgebungsverfahren nach Anspruch 12, wobei der Prozessor (350) die Position der subkutanen Struktur unterhalb einer Kontaktfläche der Ultraschallvorrichtung mit dem Körper des Subjekts auf der Grundlage der Verarbeitung eines Ultraschallenergiesignals der empfangenen Echosignale bestimmt, wobei der Prozessor mindestens eines von der Tiefe und der Abmessung der subkutanen Struktur unterhalb der Kontaktfläche auf der Grundlage einer Verarbeitung des Ultraschallenergiesignals bestimmt.

14. Bildgebungsverfahren nach Anspruch 13, wobei der Prozessor (350) das Ultraschallsignal unter Kompression der subkutanen Struktur (101C,101D) verarbeitet.

15. Bildgebungsverfahren nach einem der Ansprüche 9-14, wobei der Prozessor (350) mindestens eines von einer optimalen Nadelstärke und einem Einführwinkel für einen Zugang zu einer abgebildeten subkutanen Gefäßstruktur (101) durch eine Gefäßzugangsvorrichtung berechnet.

## Revendications

1. Dispositif à ultrasons portable (109) pour l'imagerie non invasive d'une structure sous-cutanée sélectionnée (101) chez un sujet, comprenant :
(a) un boîtier (110) ;
(b) une pluralité de réseaux (300A) d'éléments transducteurs (300), chaque réseau (300A) étant agencé en parallèle et chaque élément transducteur (300) comprenant un transducteur émetteur (705) et un transducteur récepteur (707), situés à l'intérieur dudit boîtier (110) pour transmettre en continu de l'énergie ultrasonore dans une plage de fréquences prédéterminée vers un corps (100) d'un sujet et recevoir en continu des signaux d'écho dans une plage de fréquences prédéterminée du corps (100) du sujet après réflexion de l'énergie ultrasonore, ladite pluralité de réseaux parallèles (300A) d'éléments transducteurs (300) permettant l'imagerie de la structure sous-cutanée (101) dans de multiples plans transversaux et latéraux ;
(c) un dispositif de commande (250) destiné à faire fonctionner ladite pluralité de réseaux parallèles (300A) d'éléments transducteurs (300) et pouvant communiquer avec un processeur (350) pour traiter lesdits signaux d'écho reçus à partir de ladite pluralité de réseaux parallèles (300A) d'éléments transducteurs (300) ; et
(d) un écran (104) faisant partie dudit boîtier (110) pour afficher une image interprétable (104A) de ladite structure sous-cutanée (101), dans lequel ledit processeur (350) est configuré pour traiter lesdits signaux d'écho réfléchis par la structure sous-cutanée (101) afin de produire sélectivement ladite image interprétable (104A) de la structure sous-cutanée (101) du sujet,
dans lequel le dispositif de commande (350) est configuré pour faire fonctionner ladite pluralité de réseaux parallèles (300A) d'éléments transducteurs (300) en mode Doppler à onde continue ; et
dans lequel ladite pluralité de réseaux parallèles (300A) d'éléments transducteurs (300) sont espacés les uns des autres d'une distance ξ le long d'un axe horizontal (309) sélectionné pour minimiser les interférences et maximiser une fenêtre de balayage (708), dans lequel ξ est compris entre 5 et 30 mm.

2. Dispositif à ultrasons (109) selon la revendication 1, dans lequel ladite structure sous-cutanée (101) est une structure vasculaire, éventuellement une veine.

3. Dispositif à ultrasons (109) selon la revendication 1 ou 2, dans lequel ladite pluralité de réseaux parallèles (300A) d'éléments transducteurs (300) sont inclinés selon un angle d'insonation Φ où 10 < Φ < 60.

4. Dispositif à ultrasons (109) selon l'une quelconque des revendications précédentes, dans lequel ledit écran (104) et ledit processeur (350) fournissent une indication d'un emplacement correct pour l'insertion d'un dispositif d'accès vasculaire, une ou des représentations sur l'écran (104) affichant éventuellement des informations comportant un ou plusieurs des éléments suivants : profondeur (d1-d4) de la structure sous-cutanée imagée (101) et position d'une extrémité d'aiguille insérée dans le corps du sujet.

5. Dispositif à ultrasons (109) selon la revendication 4, dans lequel des représentations en 3D sont fournies à la fois pour une structure vasculaire (101) et des champs hémodynamiques, éventuellement un ou plusieurs des éléments suivants : vitesse, pression, contrainte de cisaillement, turbulence, stagnation, pulsatilité ou sténose.

6. Dispositif à ultrasons (109) selon l'une quelconque des revendications précédentes, dans lequel ledit processeur (350) est programmé avec des instructions pour faire la distinction entre les structures vasculaires artérielles et veineuses (101, 101A) par un algorithme basé sur le temps sur la base de la mesure de la pulsatilité ; ou en raison de différences intrinsèques dans un décalage Doppler mesuré.

7. Dispositif à ultrasons (109) selon l'une quelconque des revendications 1 à 5, dans lequel ledit processeur est programmé avec des instructions pour faire la distinction entre les structures vasculaires artérielles et veineuses, dans lequel ledit processeur détermine la position de la structure sous-cutanée sous une zone de contact du dispositif à ultrasons avec le corps du sujet sur la base du traitement d'un signal d'énergie ultrasonore des signaux d'écho reçus, dans lequel le processeur détermine au moins l'une de la profondeur et de la dimension de la structure sous-cutanée sous ladite zone de contact sur la base du traitement du signal d'énergie ultrasonore.

8. Dispositif à ultrasons (109) selon la revendication 4, dans lequel le processeur (350) calcule au moins l'un d'un calibre d'aiguille optimal et d'un angle d'insertion recommandés pour l'accès à la structure vasculaire sous-cutanée imagée (101) par le dispositif d'accès vasculaire.

9. Procédé d'imagerie non invasive d'une structure sous-cutanée sélectionnée (101) chez un sujet, comprenant :
la transmission en continu d'énergie ultrasonore dans une plage de fréquences prédéterminée à un corps du sujet via une pluralité de réseaux (300A) d'éléments transducteurs (300) contenus dans un dispositif à ultrasons portable (109) appliqué au niveau ou à proximité d'un emplacement sur le corps (100) du sujet, chaque réseau (300A) de ladite pluralité de réseaux (300A) d'éléments transducteurs (300) étant agencé en parallèle et chaque élément transducteur (300) comprenant un transducteur émetteur (705) et un transducteur récepteur (707), ladite pluralité de réseaux parallèles (300A) d'éléments transducteurs (300) permettant l'imagerie de la structure sous-cutanée (101) dans de multiples plans transversaux et latéraux ;
la réception de signaux d'écho dans une plage de fréquences prédéterminée à partir du corps (100) du sujet après réflexion de l'énergie ultrasonore ;
le traitement desdits signaux d'écho reçus avec un processeur (350) ; et
la production d'une image interprétable (104A) affichant la structure sous-cutanée (101) du sujet sur un écran (104) faisant partie du dispositif à ultrasons portable (109),
dans lequel ladite pluralité de réseaux parallèles (300A) d'éléments transducteurs (300) fonctionnent en mode Doppler à onde continue ; et
dans lequel ladite pluralité de réseaux (300A) d'éléments transducteurs (300) sont espacés les uns des autres d'une distance ξ le long d'un axe horizontal (309) sélectionné pour minimiser les interférences et maximiser une fenêtre de balayage (708), ξ étant compris entre 5 et 30 mm.

10. Procédé d'imagerie selon la revendication 9, dans lequel ledit processeur (350) fait la distinction entre les structures vasculaires artérielles et veineuses (101, 101A) par un algorithme basé sur le temps sur la base de la mesure de la pulsatilité ; ou en raison de différences intrinsèques dans un décalage Doppler mesuré.

11. Procédé d'imagerie selon la revendication 9, dans lequel ledit processeur (350) fait la distinction entre les structures artérielles et veineuses (101, 101A) à l'aide d'une signature énergétique des signaux d'écho reçus pour déterminer la position d'une veine (101) sous une zone de contact (102A) du dispositif à ultrasons (109) avec le corps (100) du sujet.

12. Procédé d'imagerie selon la revendication 9, dans lequel ledit processeur (350) fait la distinction entre les structures artérielles et veineuses (101, 101A).

13. Procédé d'imagerie selon la revendication 12, dans lequel ledit processeur (350) détermine la position de la structure sous-cutanée sous une zone de contact du dispositif à ultrasons avec le corps du sujet sur la base du traitement d'un signal d'énergie ultrasonore des signaux d'écho reçus, dans lequel le processeur détermine au moins l'une de la profondeur et de la dimension de la structure sous-cutanée sous ladite zone de contact sur la base du traitement du signal d'énergie ultrasonore.

14. Procédé d'imagerie selon la revendication 13, dans lequel le processeur (350) traite le signal ultrasonore avec compression de la structure sous-cutanée (101C, 101D).

15. Procédé d'imagerie selon l'une quelconque des revendications 9 à 14, dans lequel le processeur (350) calcule au moins l'un d'un calibre d'aiguille optimal et d'un angle d'insertion pour accéder à une structure vasculaire sous-cutanée imagée (101) par un dispositif d'accès vasculaire.
